# EUROPEAN PATENT SPECIFICATION

(11) **EP 1 733 234 B1**
(45) Date of publication and mention of the grant of the patent: **14.09.2011**
(21) Application number: 05733183.7
(22) Date of filing: 22.03.2005
(51) Int. Cl.: C12Q 1/68, C12Q 1/00, B01L 3/00, G01N 33/00, G01N 15/06, G01N 33/53, G01N 33/563, G01N 33/564, G01N 33/566, G01N 33/543, C12M 1/34, G01N 33/68

(54) **METHODS OF DETERMINING ALLERGEN RESPONSE USING MICROARRAY IMMUNOASSAY TECHINQUES**
VERFAHREN ZUR BESTIMMUNG EINER ALLERGENREAKTION UNTER VERWENDUNG VON MIKROARRAY-IMMUNOASSAY-TECHNIKEN
METHODES PERMETTANT DE DETERMINER UNE REACTION ALLERGENE AU MOYEN DE TECHNIQUES DE DOSAGE IMMUNOLOGIQUE REALISES AU MOYEN DE JEUX ORDONNES DE MICROECHANTILLONS

(30) Priority: 06.04.2004 US 559825 P; 21.10.2004 US 620923 P
(43) Date of publication of application: 20.12.2006
(73) Proprietor: MOUNT SINAI SCHOOL OF MEDICINE, New York, NY 10029-6574 (US)
(72) Inventor: SAMPSON, Hugh, A., Larchmont, NY 10538 (US); SHREFFLER, Wayne, G, Bronx, NY 10029 (US); BEYER, Kirsten, 13352 Berlin (DE)
(74) Representative: Harding, Charles Thomas
(86) International application number: PCT/US2005/009567
(87) International publication number: WO 2005/100995

(56) References cited:
- EP-A1- 1 195 606
- WO-A1-02/092125
- US-B1- 6 486 311
- US-B1- 6 486 311
- JARVINEN KIRSI-MARJUT ET AL: "B-cell epitopes as a screening instrument for persistent cow's milk allergy" JOURNAL OF ALLERGY AND CLINICAL IMMUNOLOGY, MOSBY - YEARLY BOOK, INC, US, vol. 110, no. 2, August 2002 (2002-08), pages 293-297, XP002353480 ISSN: 0091-6749
- BEYER ET AL: "Measurement of peptide-specific IgE as an additional tool in identifying patients with clinical reactivity to peanuts" JOURNAL OF ALLERGY AND CLINICAL IMMUNOLOGY, MOSBY - YEARLY BOOK, INC, US, vol. 112, no. 1, July 2003 (2003-07), pages 202-207, XP005687271 ISSN: 0091-6749
- SHREFFLER W G ET AL: "Microarray immunoassay (MIA) for the parallel IgE and IgG4 epitope mapping of milk allergens." JOURNAL OF ALLERGY AND CLINICAL IMMUNOLOGY, vol. 113, no. 2 Supplement, February 2004 (2004-02), page S238, XP002449103 & 60TH ANNUAL MEETING OF THE AMERICAN ACADEMY OF ALLERGY, ASTHMA AND IMMUNOLOGY (AAAAI); SAN FRANCISCO, CA, USA; MARCH 19-23, 2004 ISSN: 0091-6749
- DEINHOFER K ET AL: "Microarrayed allergens for IgE profiling" METHODS : A COMPANION TO METHODS IN ENZYMOLOGY, ACADEMIC PRESS INC., NEW YORK, NY, US, vol. 32, no. 3, March 2004 (2004-03), pages 249-254, XP004488975 ISSN: 1046-2023
- SHREFFLER WAYNE G ET AL: "Microarray immunoassay: Association of clinical history, in vitro IgE function, and heterogeneity of allergenic peanut epitopes" JOURNAL OF ALLERGY AND CLINICAL IMMUNOLOGY, vol. 113, no. 4, 17 April 2004 (2004-04-17), pages 776-782, XP002449104 ISSN: 0091-6749

## Description

### Field of the Invention

The present invention is generally directed to methods and compositions for determining the severity of an allergic reaction.

### Background of the Invention

A large percentage of the population in North America and Europe is affected by allergic reactions to a variety of environmental allergens, making atopic disorders one the most prevalent disorders in the industrialized world (Hanson and Telemo, Acta. Pediatr. 86:916-918, 1997).

Allergic reactions are typically immune reactions initiated by IgE-dependent stimulation of tissue mast cells and related effector molecules (e.g., basophils). In such reactions, IgE molecules bind to basophils, mast cells and dendritic cells via a specific, high-affinity receptor, FcεRI (bidet, Curr. Opin. Immunol., 2:499-505,1990). When an allergen binds as a ligand to IgE, FCεRI bound to the IgE may be cross-linked on the cell surface, initiating a cascade of cellular signals in which the rapid release of biological response modifiers causes the physiological manifestations of the IgE-allergen-induced allergic reaction. These physiological effects include the release of, among other substances, histamine, serotonin, heparin, chemotactic factor(s) for eosinophilic leukocytes and/or leukotrienes C4, D4, and E4, which cause prolonged constriction of bronchial smooth muscle cells (Hood, L. E. et al., Immunology (2nd ed.), The Benjamin/Gumming Publishing Co., Inc., 1984). The clinical manifestations of these physiological effects include increased vascular permeability, vasodilation, smooth muscle contraction and local inflammation. This sequence of events is termed immediate hypersensitivity and begins rapidly, usually within minutes of exposure to the allergen in a sensitized individual.

The ultimate consequences of the interaction of an allergen with IgE are allergic symptoms triggered by release of the aforementioned mediators. Such symptoms may be systemic or local in nature, depending on the route of entry of the antigen and the pattern of deposition of IgE on mast cells or basophils. Local manifestations generally occur on epithelial surfaces at the site of entry of the allergen. In anaphylaxis, immediate, severe and systemic hypersensitivity can bring about asphyxiation, produce cardiovascular collapse, and even result in death. Individuals that are prone to strong immediate hypersensitivity responses are referred to as "atopic" and are said to suffer from "allergies." Clinical manifestations of atopy include hay fever (rhinitis), asthma, urticaria (hives), skin irritation (e.g., chronic eczema), and related conditions.

In young children, allergies to certain food products, e g., milk products, results in a failure to thrive. Allergies to various food products also severely impact the quality of life in adults. To date, a number of clinical test procedures for assessing the presence or absence of an allergic response to a given allergen have been described. See generally American College of Physicians, "Allergy Testing," Ann. Intern. Med. (1989) 110:317-320; Bousquet, J. (1988) "In Vivo Methods for Study of Allergy: Skin Tests, Techniques, and Interpretation," Allergy, Principals and Practice, 3rd ed., Middleton et al., eds., CV Mosby Co., St. Louis, Mo., pp. 419-436; and Van Arsdel et al. (1989) Ann. Intern. Med. 110:304-312. Typically, these procedures (often referred to as "skin tests" or "scratch tests") involve introducing the antigen being tested into the epidermis via a skin prick or scratch, or introducing the antigen into the dermis via intracutaneous (intradermal) injection. If the individual is sensitized to the allergen being tested, a classic atopic reaction is indicated by the occurrence of an immediate wheal and flare reaction at the site at which the allergen was introduced. When an individual is sensitized to the allergen being tested, the test site becomes red as a result of local vasodilation in the first phase of the response. In a second phase of the reaction, there appears a soft swelling (the wheal) at the site, and, in a third phase, blood vessels at the margins of the wheal dilate and become engorged with red blood cells, producing a- characteristic erythemic rim (the flare). The full wheal and flare reaction usually appears within 10 to 15 minutes of antigen administration, and generally subsides within about an hour. A wheal of sufficient size with accompanying flare represents a positive test for allergy against the antigen.

There also is accumulating data suggesting that differences in epitope recognition may be a useful prognostic marker of a patient's immune response. In the cases of certain milk (Chatchatee et al., Clin. Exp. Allergy, 31:1256-1262, 2001; Jarvinen et al., J. Allergy and Clin. Immunol, 110:293-297, 2002), egg (Cooke and Sampson, J. Immunol., 159:2026-2032, 1997) 6, and peanut allergens (Bery et al., J. Allergy and Clin. Immunol., 112:202-207, 2003), it has been shown that individuals with persistent allergy recognize a larger number of sequential epitopes, including some that appear to be specific or "informative" epitopes, as compared to those individuals who outgrow their food allergy. Specific host or stochastic factors may induce certain individuals to generate an immune response that is more broad-based or biased toward recognition of certain determinants. Even among patients with persistent allergy, analysis of the patterns of IgE epitope binding may be informative. However, little is known about the extent of diversity of epitope recognition among individual patients, what might underlie the differences that exist, what consequence such diversity may have on immune function, and how these patterns change over time.

Most of the data regarding IgE epitopes of food proteins to date have been generated through the use of the SPOTS membrane-based immunoassay (Frank and Overwin, Meth. In Mol. Biol., 66:149-169, 1996), an immunoassay that relies on the production of large numbers of arrays of synthetic peptides prepared on cellulose membranes. However, for the characterization of large numbers of individual patient allergen-specific IgE antibodies, this method has technical disadvantages. Synthesis of large numbers of peptides is relatively time consuming, labor intensive, expensive and error-prone. The SPOTS immunoassays require milliliter quantities of patient serum and although individual membranes can be stripped, they can only be reused for a limited number of patients. For these reasons, identification of epitopes is often performed with pooled sera and evaluation of individual patients has generally been limited. These technical considerations affect the experimental design since it is impractical to assay multiple controls in order to define positive cutoff values, or to perform replicates to determine assay variance.

Despite the wide-spread availability of various diagnostic tests, these tests are inadequate for evaluating the degree and severity of an allergic reaction. In the case of the skin prick tests, such diagnostics are merely predictive of the presence or absence of an IgE-mediated response against a given antigen. An immune response to any given allergen in an individual can vary from being a mild, yet tolerable response that produces some discomfort, to a severe and life-threatening anaphylactic shock. In the case of SPOTS analysis, there is a need to produce large quantities of allergen proteins for the membranes and to use large amounts of serum sample. As the serum samples are typically pooled, an individual response is difficult to assess. Therefore, the tests available to date fail to predict the severity or degree of such a response and a need remains for new diagnostic tests for determining the severity of an immune response.

J Allergy Clin. Immunol. 112 (1):202-207 (2003) discloses that specific immunodominant epitopes are associated with IgE binding in peanut allergy patients but not tolerant patients.

### SUMMARY OF THE INVENTION

The present invention provides a method of predicting the severity of an immunological response against an allergen in a subject, wherein a mild immunological response involves cutaneous symptoms and a severe immunological response involves multiple organ systems, the method comprising binding IgE from the serum of said subject to a plurality of epitopes from an allergen and determining the degree of epitope binding diversity of the IgE from the serum of said subject to a plurality of epitopes on said allergen, wherein IgE binding to four or more epitopes on said allergen indicates a severe allergic response, wherein IgE binding to less than four epitopes indicates a mild allergic response, and wherein the allergen is a food allergen responsible for IgE-mediated hypersensitivity.

In one embodiment, the epitopes from said allergen are arrayed on a solid support.

In one embodiment, said binding of said IgE from the serum of said subject is carried out on a microarray immunoassay.

In one embodiment, said allergen is a protein.

In one embodiment, the epitopes from the protein are arrayed on a solid support as a series of overlapping peptide fragments of said allergen.

In one embodiment, said IgE from the serum of said subject binds to sequential epitopes on said allergen.

In one embodiment, said food allergen is an allergen from a foodstuff optionally selected from the group consisting of a legume, a tree nut, a cereal grain, a fruit, a vegetable, a seed, a fish, a crustacean, a mollusc, poultry and a dairy product.

In one embodiment, said allergen is a peanut allergen selected from the group consisting of Ara h1, Ara h2 and Ara h3.

In one embodiment, said binding of said IgE from the serum of said subject to said peanut allergen is carried out using a microarray immunoassay, wherein said microarray comprises an array of overlapping peptides from one or more of the allergens Ara h1, Ara h2, and Ara h3.

In one embodiment, said overlapping peptides each comprise an amino acid sequence of from 10 amino acids in length to 30 amino acids in length.

In one embodiment, each of said overlapping peptides comprises a sequence that overlaps with at least 50% of the sequence of at least one other peptide in said microarray.

In one embodiment, said microarray comprises an array of peptides 20 amino acids in length derived from Ara h1, Ara h2, or Ara h3, wherein each peptide has a 15 amino acid overlap with at least one other peptide derived from the same allergen.

In one embodiment, said determining comprises an immunoassay which comprises contacting a microarray of a plurality of sequential epitopes from said allergen with serum obtained from said subject and determining the amount of IgE bound to each epitope in said microarray using an anti-IgE antibody.

In one embodiment, said microarray comprises epitopes derived from a plurality of different allergens.

In one embodiment, said different allergens are derived from the same source.

In one embodiment, said different allergens are derived from different sources.

In one embodiment, the allergen is a peanut allergen and the plurality of epitopes comprises an isolated peptide of residues 361-385 of SEQ ID NO:13.

In one embodiment, predicting the severity of an immunological response against an allergen in a subject comprises predicting an anaphylactic reaction against an allergen in the subject.

### BRIEF DESCRIPTION OF THE DRAWINGS

The following drawings form part of the present specification and are included to further illustrate aspects of the present invention. The invention may be better understood by reference to the drawings in combination with the detailed description of the specific embodiments presented herein.

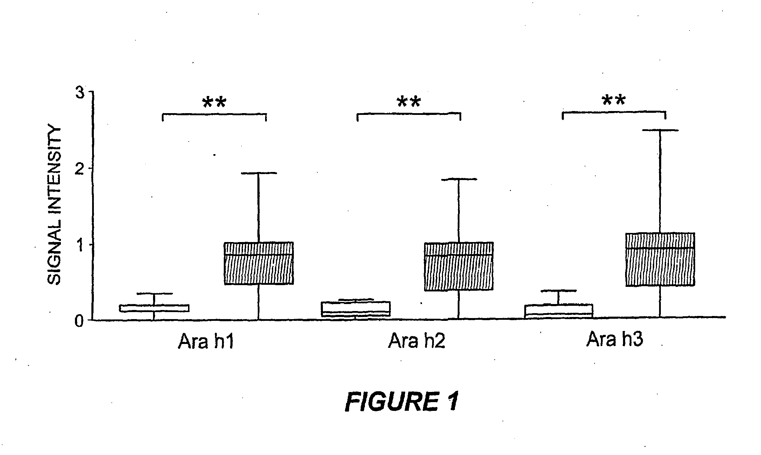

**Figure 1****.** The microarray immunoassay detects allergen-specific IgE. Peanut-sensitized patients (n=77, shaded boxes) versus controls (n=15, open boxes) have significantly higher levels of IgE as detected by this assay to rAra h1, rAra h2, and rAra h 3 (p<0.001 for each comparison between patient groups). Signal intensity is the ratio of protein signal to positive control signal on a log scale (see Example 1).

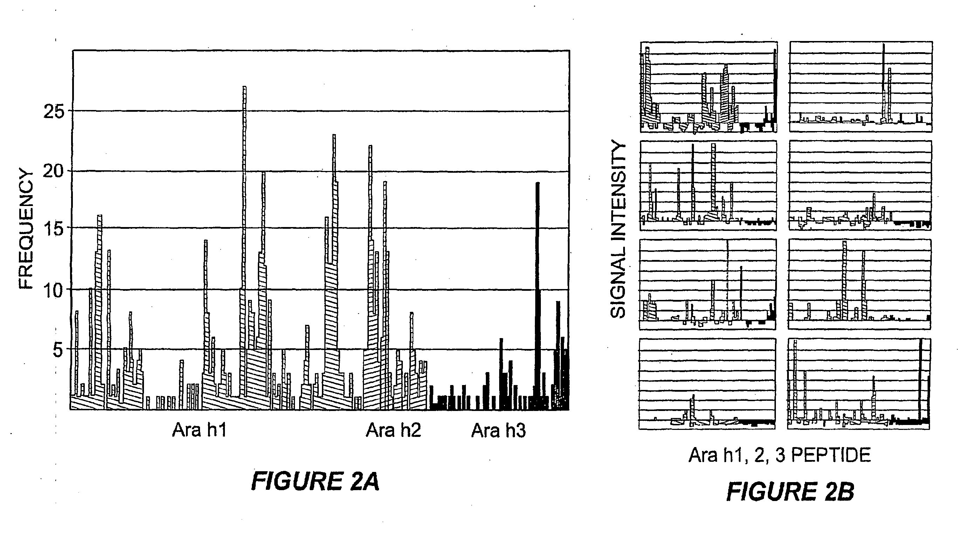

**Figure 2A and Figure 2B****.** IgE epitope profiles: Figure 2A shows the frequency of recognition plotted against each peptide on the microarray ordered from N to C terminus of Ara h 1, Ara h 2, and Ara h 3 for a group of 77 peanut-sensitized patients. Figure 2B. Eight individual patient IgE profiles are shown with signal intensity plotted against peptide to demonstrate the heterogeneity of IgE epitope recognition.

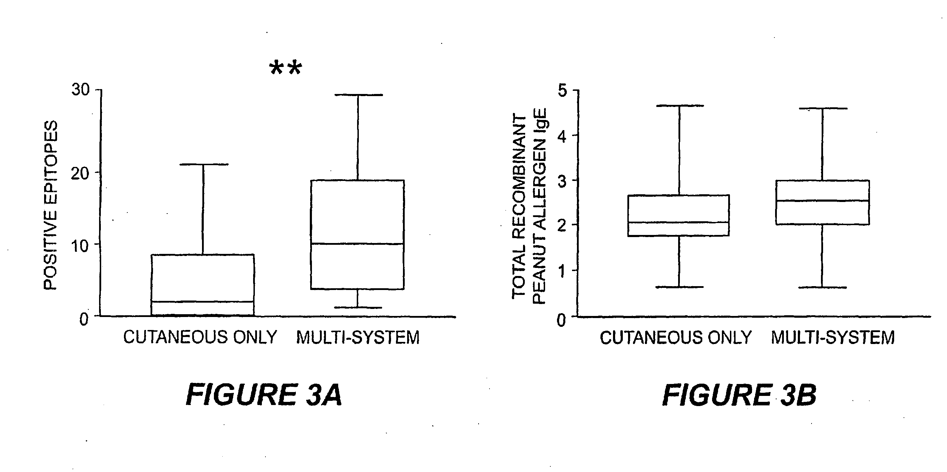

**Figure 3A and Figure 3B****.** Individuals with a history of more severe peanut-induced reactions recognize more peanut allergen epitopes. Figure 3A. Individuals with a history of cutaneous symptoms (n=14) recognize fewer epitopes (median 2, range 0-20) compared to those with a history of reactions involving multiple organ systems (n=24, median 9.5, range 1-29; p<0.01). Figure 3B. The same patients have similar recognition of recombinant peanut allergens.

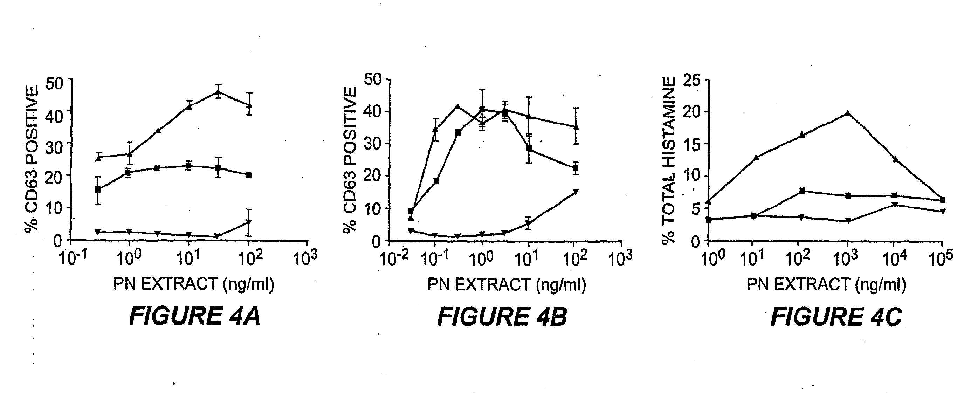

**Figure 4A****-****Figure 4C****.** Patient sera recognizing a greater number of IgE epitopes induce more potent degranulation of passively sensitized cells. Pooled sera from patients with high levels of peanut-specific IgE (>100 kU/L, n=4 for each group) were generated from sera recognizing few epitopes (square symbols) vs. individuals' sera recognizing many epitopes (triangles), and sera from non-peanut allergic controls (diamond). Serum pools were used to passively sensitize human basophils from a non-atopic donor (Figure 4A), an atopic, non-peanut allergic donor (Figure 4B) and the rat basophilic leukemia cell line expressing the human FcεRI (Figure 4C). With all donor cells, the sera containing more diverse IgE epitope recognition conferred greater degranulation when challenged with peanut antigen.

### DESCRIPTION OF THE PREFERRED EMBODIMENTS

Food allergy affects 6% of children younger than 4 years in age and about 2% of the U.S. population beyond the first decade of life. It is the single leading cause of anaphylaxis treated in hospitals in the Industrialized world. Despite the availability of certain diagnostic tests, there remains a need to be able to predict the severity of an immune response of an individual to a given allergen(s).

### 1. DEFINITIONS

An "immunological response" or "immune response" against a selected agent or a composition of interest is the development in an individual of a humoral and/or a cellular immune response to molecules (e.g., antigens or allergens) present in the agent or composition of interest. A brief overview of development of an immune response as it relates to allergies is provided in further detail below in Section II.

An "antigen refers to any immunogenic moiety or agent, generally a macromolecule, which can elicit an immunological response in an individual. The term may be used to refer to an individual macromolecule or to a homogeneous or heterogeneous population of antigenic macromolecules. An "allergen" is an antigen which can initiate a state of hypersensitivity, or which can provoke an immediate hypersensitivity reaction in an individual already sensitized with the allergen. Allergens are commonly proteins or chemicals bound to proteins which have the property of being allergenic; however, allergens can also include organic or inorganic materials derived from a variety of man-made or natural sources such as plant materials, metals, ingredients in cosmetics or detergents, latexes, or the like. Allergens can elicit any type of hypersensitivity reaction in a sensitized individual. Exemplary allergens are discussed in further detail in Section III below.

The term "epitope diversity" of an IgE sample as used herein refers to the number of epitopes that the IgE recognizes.

The terms "individual" and "subject" are used interchangeably herein to refer to any member of the subphylum cordata, including, without limitation, humans and other primates, including non-human primates such as chimpanzees and other apes and monkey species; farm animals such as cattle, sheep, pigs, goats and horses; domestic mammals such as dogs and cats; laboratory animals including rodents such as mice, rats and guinea pigs; birds, including domestic, wild and game birds such as chickens, turkeys and other gallinaceous birds, ducks, geese, and the like. The terms do not denote a particular age. Thus, both adult and newborn individuals are intended to be covered. The methods described herein are intended for use in assessing the severity of an allergic response in any of the above vertebrate species, since the immune systems of all of these vertebrates operate using similar principles.

### II. DEVELOPMENT OF FOOD AND ENVIRONMENTAL ALLERGIES

In order to provide a better understanding of the relevance of the diagnostic tests of the present invention, the present section provides a brief overview of the development of allergies in a subject.

IgE-mediated hypersensitivity mechanisms account for the majority of food-related allergic disorders. For example, approximately 2.5% of infants are born with an allergy to milk. 15% of infants with IgE-mediated cow milk allergy retain mil product sensitivity into the second decade, 35% develop allergic reactions to other foods, and about 60% develop respiratory allergy. The increased susceptibility of young infants to food allergic reactions is believed to result from immaturity of the immune system and gastrointestinal tract. The gastrointestinal tract forms a barrier to the outside environment and provides a surface to process and absorb ingested food, and to discharge waste products. The immune system associated with this barrier, the gut-associated lymphoid tissue [GALT], is capable of discriminating between harmless foreign proteins and commensal organisms, and dangerous pathogens.

The mucosal immune system is comprised of both the innate and adaptive immune systems. Gut epithelial and mucosal cells, which form a major portion of the mucosal innate immune system, are well suited to initiate protective responses. Unlike the systemic immune system, the adaptive mucosal immune system is specifically proficient at inhibiting responses to non-dangerous antigens [oral tolerance] and yet mounting a rapid response to pathogens. However, developmental immaturity of various components of the gut barrier [e.g., decreased stomach acidity and intestinal and pancreatic enzyme activity, immature glycocalyx, and the like] and immune system reduces the efficiency of the infant mucosal barrier, and likely plays a major role in the increased prevalence of gastrointestinal infections and food allergy seen in the first few years of life. The early introduction of numerous food antigens has been shown to stimulate excessive production of IgE antibodies (Soothill et al., Clin Allergy, 6:305-319, 1976) and induce allergic conditions in genetically predisposed infants.(Fergusson et al., Pediatrics 86:541-546, 1990).

The GALT is comprised of four distinct lymphoid compartments: Peyer's patches and the appendix, lamina propria lymphocytes and plasma cells, intra-epithelial lymphocytes, and mesenteric lymph nodes (Mayer et al., J. Pediatr. Gastroen. Nutri, 30:S4-S12,2000). M cells, specialized epithelial cells overlying Peyer's patches, sample antigens [e.g., foreign proteins] from the gut lumen, especially large particulate antigens and a number of bacteria and viruses, and pass them intact to underlying macrophages. Macrophages process and present these antigens to resident precursor T cells and B cells, which become activated and then "home" to other areas of the mucosal immune system. Local B cells produce secretory-IgA antibodies [S-IgA], which are actively transported into intestinal secretions forming a component of the protective barrier (Mestecky et al., Immunol Allergy Clin N Am, 8:349-368, 1988).

Despite the evolution of an elegant barrier system, about 2% of ingested food antigens are absorbed and transported throughout the body in an "immunologically" intact form, even through the mature gut (Husby et al., Gut, 28:1062-1072, 1987). Recent studies in rodent models have demonstrated the presence of FcεII receptors on gut epithelial cells that facilitate antigen-specific transport [10-times faster] in IgE-sensitized rats as compared to non-sensitized controls (Berin et al., J Immunol., 161:2561-2566, 1998; Yang et al., J Clin Invest., 106(7):879-886, 2000).

As noted, intact food antigens penetrate the gastrointestinal tract and enter the circulation in both normal children and adults. However, these intact proteins generally do not cause clinical symptoms because most individuals develop tolerance to ingested antigens. In mucosal tissues, soluble antigens, such as food antigens, are typically poor immunogens and induce a state of unresponsiveness known as "oral tolerance." Unresponsiveness of T cells to ingested food proteins is believed to be the result of T cell anergy and/or induction of regulatory T cells. Intestinal epithelial cells [IECs] play a major role in tolerance induction to food antigens, acting as non-professional antigen presenting cells (Mayer et al., J. Pediatr. Gastroen. Nutri, 30:S4-S12, 2000). In addition, dendritic cells residing within the non-inflammatory environment of Peyer's patches express IL-10 and IL-4, which favor generation of tolerance. T regulatory cells [Th3 and Tr-1 cells], which are potent sources of TGF-β (Smith et al., Am J Respir Crit Care Med., 162(4 Pt 2):S175-S 178, 2000) are generated in mucosal lymphoid tissue in response to low-dose antigen and mediate "bystander tolerance" within the GI tract. The gut flora also is believed to play a significant role in the induction of oral tolerance since animals raised in a germ-free environment from birth fail to develop normal tolerance (Sudo et al., J. Immunol., 159(4):1739-1745, 1997). The generation of regulatory T cells appears to require a threshold level of inflammatory response to bacterial challenge (Murch, Lancet, 348(9042):1656, 1996)

The development of an IgE-mediated response to an allergen [generally a glycoprotein] is the result of a series of molecular and cellular interactions involving antigen-presenting cells [APCs], T cells, and B cells. APCs [dendritic cells, activated B cells] present small peptide fragments [T cell epitopes] in conjunction with MHC class II molecules to T cells. T cells bearing the appropriate complementary T cell receptor [TCR] will bind to the peptide-MHC complex. This interactive "first signal" leads to T cell proliferation and cytokine generation and the generation of a "second" signal, which promotes an IgE response [Th2 lymphocyte activation]. When antigen becomes bound to surface immunoglobulin on B cells, the cell is activated and processes antigen for expression of peptides bound to MHC class II molecules. T cells and their products will in turn interact with B cells bearing appropriate antigen-specific receptors leading to isotype switching and the generation of antigen-specific IgE. At all stages, a number of specific cytokines are secreted which modulate the cell interactions. The antigen-specific IgE then binds to surface receptors of mast cells, basophils, macrophages, and other APCs, arming the immune system for an allergic reaction with the next encounter of the specific antigen.

The next and subsequent encounters of the specific antigen trigger an immunological cascade that results in the manifestations of an allergic response. In some individuals, the subsequent responses may be severe, even to the level of being threatening; in other individuals, the response may be less severe. The present invention is designed to prognostically predict the level of severity of an immune response by assessing the degree of epitope diversity that the IgE of the subject exhibits. As described in the examples herein below, it has been determined that the greater the degree of epitope diversity against any given allergen, the greater the severity of the immune response.

### III. A DIAGNOSTIC TEST FOR DETERMINING SEVERITY OF ALLERGIC RESPONSE

The present invention is preferably directed to a microarray-based immunoassay (MIA) that allows parallel analysis of IgE binding to a set of overlapping peptides from the sequences of various dietary allergens using microliter amounts of patient serum per assay. Using this assay, the degree of heterogeneity among sensitized individuals with respect to their epitope binding patterns reveals important diagnostic and prognostic information about the severity of allergic response that the individual will suffer when exposed to that allergen. Greater diversity of peptide epitope recognition correlates only weakly with allergen-specific IgE levels (i.e., amount of KU/L). However, patients with IgE binding to a greater number of epitopes have a history of more severe allergen-induced allergic reactions. In addition, epitope diversity corresponds with functional capacity to induce activation of FcεRI-bearing cells in response to allergen, but not to anti-IgE levels.

In the present methods, a microarray of epitopes from one or more allergens is prepared as described herein below. Serum samples from the subject to be tested are obtained using techniques known to those of skill in the art. The subject may be any subject for whom an allergic response is to be determined. Preferably the subject is a mammal. More preferably, the subject is a human; however, the methods of the invention may be applicable in veterinary diagnostics of allergies that domestic and farm animals may be suffering and, as such, animals such as cats, dogs, cows, horses and mammals in a zoo may be "subjects" in the context of the present invention. The serum samples collected from the subject of interest are applied to the microarray and the binding of the IgE from the serum to the epitopes arrayed on the microarray is determined. In exemplary embodiments, the determination of the binding of serum IgE is assessed using an immunoassay in which an antibody that recognizes IgE is used.

In exemplary immunoassays of the invention, a variety of peptides are immobilized onto a selected surface as a microarray as described herein below. The surface may be any surface onto which a protein may be adhered. As such, the surface may include a microchip or even the wells of a microtiter plate. In preferred embodiments, the surface is a poly-L-lysine coated microscope slide. In some embodiments, a solid surface may be coated with a non-specific protein that is known to be antigenically neutral with regard to the test antibody. This will allow for blocking of nonspecific adsorption sites on the immobilizing surface, and thus reduce the background caused by nonspecific binding of antisera onto the surface.

Next, the serum from the test individual is added to the plate in a manner conducive to allow an immune complex (antigen/antibody) formation between the peptides presented on the solid surface and IgE molecules present in the serum of the test subject. The microarray containing the subject serum is allowed to incubate for about 1 hour to about 4 hr, at temperatures preferably on the order of about 25° to about 27°C (i.e., typical room temperature), however, it should be understood that the incubation may be carried out at other temperatures. In a preferred embodiment, the microarray is incubated for about 4 hours. Following incubation, the plate is washed to remove non-immunocomplexed material.

Following formation of specific immunocomplexes between the arrayed peptides and the IgE from the sera, and subsequent washing, the occurrence and amount of immunocomplex formation may be determined by contacting the plate with an antibody having specificity for, and therefore recognizing, IgE as a target. To provide for detection of the bound complex, the second antibody will preferably be associated with an enzyme that will generate a detectable color or other signal upon incubating with an appropriate substrate, such as a chromogenic substrate. Thus, for example, one will contact and incubate the antibody-bound surface with a urease, alkaline phosphatase or peroxidase-conjugated anti-IgE antibody for a period of time and under conditions that allow the development of immunocomplex formation (e.g., incubation for 2 hr at room temperature in a PBS-containing solution such as PBS/Tween®. Other conjugated antibodies also are known and suitable. In addition, rather than being labeled with an enzyme, the antibody may be conjugated to the substrate of the enzyme.

After incubation with the anti-IgE enzyme-tagged antibody, and subsequent to washing to remove unbound material, the amount of label bound to the IgE is quantified by incubation with a chromogenic substrate such as urea and bromocresol purple or 2,2'-azino-di-(3-ethyl-benzthiazoline)-6-sulfonic acid (ABTS) and H₂O₂ in the case of peroxidase as the enzyme label. Quantitation is then achieved by measuring the degree of color generation, e.g., using a visible spectrum spectrophotometer.

In still other embodiments, the detection is facilitated through the use of a radio-, fluorescent, or other chromogenic label attached to the anti-IgE antibody. Such labels include but are not limited to labels that can be detected by spectroscopic, photochemical, biochemical, bioelectronic, immunochemical, electrical, optical or chemical methods. Radiolabels would include e.g., radioisotopes, such as ³H, ¹⁴C, ³²P_{,} ³³P or ³⁵S; chemiluminescent compounds, labeled binding proteins, heavy metal atoms, spectroscopic markers such as fluorescent markers and dyes, magnetic labels, linked enzymes, mass spectrometry tags, spin labels, electron transfer donors and acceptors, and the like also may be used.

Anti-IgE antibodies for use in such immunoassays to detect the presence of IgE are well known to those of skill in the art and commercially available, and may be polyclonal antibodies or may be monoclonal antibodies. Exemplary antibodies include e.g., ab4221 (rabbit polyclonal antibody generated against human IgE antibody); ab7294 (mouse monoclonal antibody generated against human IgE antibody [4F4]); ab773 (mouse monoclonal human IgE antibody [4G7]; ab774 (mouse monoclonal antibody generated against human IgE antibody [4H10]); ab7295 (mouse monoclonal antibody generated against human IgE antibody [5D4]; ab775 (mouse monoclonal antibody generated human IgE antibody [BE5]); ab7378 (mouse monoclonal antibody generated human IgE antibody [E411]; ab7382 (mouse monoclonal antibody generated human IgE antibody [XTE4]) all available from Abcam Ltd., Cambridge, MA. Other anti-IgE antibodies are available from BD Biosciences Pharmingen (San Diego, CA), e.g., catalog # 555894 (mouse monoclonal anti-human IgE antibody); catalog # 555857 (mouse monoclonal anti-human IgE antibody); catalog # 555858 (biotin conjugated mouse monoclonal anti-human IgE antibody); catalog # 555859 (alkaline phosphatase-conjugated mouse monoclonal anti-human IgE antibody); catalog # 553419 (biotin conjugated rat monoclonal anti-human IgE antibody); catalog # 553413 (rat monoclonal anti-human IgE antibody); catalog # 553416 (rat monoclonal anti-human IgE antibody); catalog # 553914 (mouse monoclonal anti-human IgE antibody); catalog # 553916 (biotin conjugated mouse monoclonal anti-human IgE antibody); Other similar antibodies generated from various mammals, including sheep, rat, mice, goats and the like are variously available from, e.g., Beckman Coulter (Fullerton, CA); BIODESIGN International (Saco, Maine); Biosource International (Camarillo California), Calbiochem (San Diego, CA); CalTag (Burlingame, CA); Chemicon; (Temecula, CA), eBioscience (San Diego, CA); KPL, Inc., (Gaithersburg, Maryland);Leinco Technologies, Inc. (St. Louis, MO); Maine Biotechnology Services, Inc. (Portland, Maine); Novus Biologicals (Littleton, CO); and Serotec Inc. (Raleigh, NC).

Other immunoassays encompassed by the present invention include, but are not limited to, those described in U.S. No. Patent 4,367,110 (double monoclonal antibody sandwich assay).

While in a preferred embodiment the present invention employs microarrays of immobilized peptides derived from an allergen that are contacted with a solution of serum from the subject to be tested, the converse also is contemplated. In such embodiments, the serum is coated onto a solid surface, e.g., into a multiwell plate, and allergen-derived peptides are dispensed onto each of the surfaces containing the serum. Unbound peptide is removed by washing of the plates and the amount of peptide bound to the serum is quantified. Such quantification may be facilitated by the presence of a label on each of the peptides. Labeling can be carried out during the synthesis of the peptide or the label may be incorporated after the peptide has been synthesized. Examples of labels include spectroscopic, photochemical, biochemical, bioelectronic, immunochemical, electrical, optical or chemical labels. The labeling moieties include radioisotopes, such as ³H, ¹⁴C, ³²P, ³³P or ³⁵S; chemiluminescent compounds, labeled binding proteins, heavy metal atoms, spectroscopic markers such as fluorescent markers and dyes, magnetic labels, linked enzymes, mass spectrometry tags, spin labels, electron transfer donors and acceptors, and the like.

Exemplary dyes include quinoline dyes, triarylmethane dyes, phthaleins, azo dyes, cyanine dyes, and the like. Preferably, fluorescent markers absorb light above about 300 nm, preferably above 400 nm, and usually emit light at wavelengths at least greater than 10 nm different from the wavelength of the light absorbed. Preferred fluorescent markers include fluorescein, phycoerythrin, rhodamine, lissamine, and C3 and C5 available from Amersham Pharmacia Biotech (Piscataway N.J.), Alexa 546, and Alexa 647 (Molecular Probes, Eugene OR).

Using the assays of the present invention, it is contemplated that those skilled in the art will be able to predict the relative severity of an allergic response of the subject to a given allergen. More particularly, it is shown herein that the greater the diversity of IgE binding from the serum of a subject to the number of epitopes it encounters on any given allergen, the more severe the allergic response is in that individual. Thus, to determine the severity of an allergic response to a given allergen, the skilled artisan will determine the degree of epitope binding diversity of the IgE from the serum of the subject to a plurality of epitopes on the allergen. It is contemplated that where the IgE binds to four or more epitopes on any given allergen, the subject's allergy to that allergen is severe. The epitopes may be sequential from the amino acid structure of the allergen or the epitopes may be non-sequential. In preferred embodiments, it is contemplated that 4, 5, 6, 7, 8, 9, 10, 11, 12, 13, 14, 15, 16, 17, 18, 19, 20, 21, 22, 23, 24, 25, 26, 27, 28, 29, 30, or more sequential or non-sequential epitopes from the same allergen will be indicate that the individual has a severe allergic response to that allergen. In specific embodiments, it is contemplated that the epitopes will typically be in the range of from about 8 amino acids to about 20 amino acids in length.

Typically, the epitopes described herein are peptides that are between 5 amino acids and 50 amino acids in length. More preferably, the epitope-bearing peptides are between 8 and about 25 amino acids in length and more preferably, in the range of from about 8 amino acids to about 20 amino acids in length. For example, it is expressly contemplated that the epitope-bearing peptides are 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or more amino acids in length. Preferably, the epitopes are between 10 to 25 amino acids in length. In an array as described further herein below, a plurality of such epitopes are arrayed. Each of the epitopes preferably overlaps substantially with at least one other epitope in the array. For example, an array is made from a hypothetical protein N amino acids in length (A¹-A^{N}), and divided into allergen epitopes as follows Allergen: A¹A²A³A⁴A⁵A⁶A⁷A⁸A⁹A¹⁰A¹¹A¹²A¹³A¹⁴A¹⁵ A¹⁶A¹⁷A¹⁸A¹⁹A²⁰A²¹A²²A²³A²⁴A^{25...}A^{N}; epitopes: Epitope 1: A¹-A²⁰; Epitope 2: A²-A²²; Epitope 3: A⁴-A²⁴; Epitope 4: A⁶-A²⁶; Epitope 5: A⁸-A²⁸; in like manner, epitopes of a given length, (20 amino acids in this example, but any length as discussed above) may be generated from the entire span of the allergen from A¹ through A^{N} and used in the assays described herein. The overlaps in this example are 18 amino acids in length; however, it is contemplated that the overlaps may be of 3, 4, 5, 6, 7, 8, 9, 10, 11, 12, 13, 14, 15, 16, 17, 18, 19, 20, 21, 22, 23, 24, 25, 26, or more amino acids in length depending on the size of the epitope being used. Preferably the overlap between sequential epitopes is at least 50% of the previous epitope (e.g., in the above example if the first epitope is A¹-A²⁰, the second sequential epitope should preferably be at least A¹⁰-A³⁰, and the third sequential epitope should preferably be at least A²⁰-A³⁰). Preferably, the overlap is between 50% and 90%, as such the overlap may be 60%, 65%, 70%, 75%, 80%, 85%, 90% etc. However, the size of the epitope and the degree of overlap may be varied according to particular needs using routine experimentation.

In addition, while the above discussion has focused on generally describing epitopes from one allergen, it should be understood that the methods of the present invention will typically employ epitopes from multiple allergens from the same allergen source (e.g., a particular foodstuff) or alternatively may employ multiple epitopes from multiple allergens from a plurality of sources (e.g., multiple foodstuffs.)

Using the methods of the present invention, it has been determined that subjects having a low overall allergen-specific IgE level (e.g., approx. 1-2kU/L) may be more severely allergic to an allergen than subjects producing a higher overall allergen-specific IgE level (e.g., 10kU/L or more), where the low overall IgE level is generated as a result of recognition of more epitopes than the higher IgE response. As such, the methods of the present invention are advantageous over the traditional skin-prick tests which may yield a false negative overall result with respect to a given allergen because such tests will primarily assess the presence or absence of a particular immune response and that presence or absence typically will quantitatively correlate proportionally with the level of allergen-specific IgE (in terms of kU/L) measured. The methods of the present invention on the other hand are able to convert the quantitative response into a qualitative determination of whether or not the allergen will produce a more or less severe allergic reaction depending on the number of epitopes of that allergen that are recognized by the IgE in the serum of the test subject.

### IV. ALLERGENS FOR USE IN DIAGNOSTIC ASSAYS

As discussed above, the methods of the present invention allow a prediction of the severity of an allergic response that an individual will suffer. These methods will employ epitopes derived from any allergens to be tested, provided that the allergens are food allergens.

Thus, the methods for assessing the severity of an allergic response that are described herein can be carried out using any one or more suspected allergens, the selection of which will generally be dictated by the test parameters. For example, these methods can be used to confirm the presence of immediate-type hypersensitivity to allergens suspected from an individual's history or to confirm allergies to commonly encountered allergens such as a variety of foods; or to document immediate hypersensitivity prior to conducting other allergy testing, such as provocation testing (bronchial provocation, oral food provocation), or prior to allergy desensitization therapy.

Allergens from numerous foodstuffs are well known to those of skill in the art. Amongst common foodstuffs that produce an allergic response in subjects are foods such as dairy products, nuts, fruits, fish, shellfish and the like. Typical foods in which the allergens have been characterized include kiwi; cashew; pineapple; celery; peanut; brazil nut; european chestnut; carrot; soybean; barley; English walnut; tomato; apple; banana; rice; avocado; apricot; cherry; plum; peach; pear; strawberry; sesame; white mustard; potato; corn; bovine; crab; American oyster; carp; Baltic cod; chicken; snail; American lobster; shrimp; scallop; lobster; mussel; salmon. The allergens that have been characterized for these foodstuffs are identified in Table 1 below.

**Table 1 list of common food allergens**

| **Plant Allergen** | **Common Name** | **Allergen Name** | **Type of Protein** | **Amino Acid Length** |
|---|---|---|---|---|
| Actinidia chinensis | Kiwi | Act c 1 | Actinidain (Actinidin) | 380 |
| Anacardium occidentale | Cashew | Ana o 1.0101 | Vicilin-like protein | 538 |
| Ananas comosus | Pineapple | Ana c 1 | Profilin | 131 |
| Apium graveolens | Celery | Api g 4 | Profilin | 134 |
| Apium gaveolens | Celery | Api g 1 | | 154 |
| Arachis hypogaea | Peanut | Ara h 3 | Glycinin | 507 |
| Arachis hypogaea | Peanut | Ara h 4 | Glycinin | 530 |
| Arachis hypogaea | Peanut | Ara h 5 | Profilin | 131 |
| Arachis hypogaea | Peanut | Ara h 6 | Conglutin-like | 129 |
| Arachis hypogaea | Peanut | Ara h 7 | Conglutin-like | 160 |
| Arachis hypogaea | Peanut | Ara h 1 | Vicillin | 614 |
| Arachis hypogaea | Peanut | Ara h 2 | Conglutin | 156 |
| Bertholletia excelsa | Brazil nut | Ber e 1 | 2S sulfur-rich seed storage protein | 146 |
| Castanea sativa | European chestnut | Cas s 1 | Pathogenesis-related protein family 10 | 160 |
| Daucus carota | Carrot | Dau c 4 | Profilin | 134 |
| Daucus carota | Carrot | Dau c 1.0103 | Bet v 1 homolog | 154 |
| Glycine max | Soybean | Gly m 2 | Hull protein (fragment), airway allergen | 20 |
| Glycine max | Soybean | Gly m3 | profilin | |
| Glycine max | Soybean | Gly m4 | Starvation-associated message 22 | |
| Glycine max | Soybean | Gly m 1.0101 | Hydrophobic seed protein (fragment) | 42 |
| Hordeum vulgare | Barley | Hor v 15 | Alpha-amylase inhibitor BMAI-1 | 146 |
| Juglans regia | English walnut | Jun r 1 | Albumin seed storage protein | 139 |
| Juglans regia | English walnut | Jug r 2 | Vicilin-like protein | 593 |
| Lycopersicon esculentum | Tomato | Lye e 1 | Profilin | 131 |
| Lycopersicon sculentum | Tomato | Lye e 2.0102 | Beta-fructofuanosidase | 636 |
| Malus x domestica | Apple | Mal d 1 | | 159 |
| Malus x domestica | Apple | Mal d 3 | Lipid transfer protein | 115 |
| Malus x domestica | Apple | Mal d 2 | Thaumatin-like protein | 246 |
| Musa acuminata | Banana | Mus xp 1 | Profilin | 131 |
| Oryza sativa | Rice | Ory s.1 | Expansin-like protein | 263 |
| Persea americana | Avocado | Pers a 1 | Endochitinase | 326 |
| Prunus armeniaca | Apricot | Pru ar 1 | | 160 |
| Prunus armeniaca | Apricot | Pru ar 3 | Nonspecific lipid-transfer protein 1 | 91 |
| Prunus avium | Cherry | Pru av 2 | Thaumatin-like protein | 245 |
| Prunus avium | Cherry | Pru av 1 | Bet v 1 -like protein | 160 |
| Prunus avium | Cherry | Pru av 4 | Profilin | 131 |
| Prunus avium | Cherry | Pru av 3 | Lipid transfer protein | 117 |
| Prunus domestica | Plum | Pru d 3 | Nonspecific lipid-transfer protein 1 | 91 |
| Prunus persica | Peach | Pru p 3 | Nonspecific lipid-transfer protein 1 | 91 |
| Pyrus communis | Pear | Pyr c 1 | Bet v 1-like protein | 159 |
| Pyrus communis | Pear | Pyr c 1 | Profilin | 131 |
| Sesamum indicum | Sesame | Ses i 2 | 2S albumin | 148 |
| Sesamum indicum | Sesame | Ses i 1 | 2S albumin precursor | 153 |
| Sesamum indicum | Sesame | Ses i 3 | 7S globulin | 585 |
| Sinapis alba | White mustard | Sin a 1 | Amylase inhibitor | 145 |
| Solanum tuberosum | Potato | Sola t 2 | Aspartic protease inhibitor 11 | 188 |
| Solanum tuberosum | Potato | Sola t 3 | Cysteine protease inhibitor 1 | 222 |
| Solanum tuberosum | Potato | Sola t 4 | Serine protease inhibitor 7 | 221 |
| Solanum tuberosum | Potato | Sola t 1 | Patatain B1 | 377 |
| Zea mays | Corn | Zea m 14 | Nonspecific lipid-transfer protein | 120 |
| | | | | |

| **Animal Allergen** | **Common Name** | **Allergen Name** | **Type of Protein** | **Amino Acid Length** |
|---|---|---|---|---|
| Bos taurus | Bovine | Bos d 6 | Albumin | 607 |
| Bos taurus | Bovine | Bos d 4 | Alpha-lactalbumin | 142 |
| Bos Taurus | Bovine | Bos d 8 | Alpha-s1-casein | 214 |
| Bos Taurus | Bovine | Bos d 8 | Beta-casein | 224 |
| Bos Taurus | Bovine | Bos d 8 | Kappa-casein | 190 |
| Bos Taurus | Bovine | Bos d 8 | Alpha-s2-like casein | 222 |
| Bos taurus | Bovine | Bos d 5 | Alpha-s2-like casein | 178 |
| Charybdis feriatus | Crab | n/a | Heat stable tropomyosin | 264 |
| Crassostrea gigas | America oyster | n/a | Tropomyosin | 233 |
| Cyprinus carpio | Carp | n/a | Parvalbumin | 109 |
| Gadus callarias | Baltic cod | Gad c 1 | Parvalbumin beta | 113 |
| Gallus gallus | Chicken | Gad d 2 | Ovalbumin | 388 |
| Gallus gallus | Chicken | Gad d 1 | Ovomucoid | 210 |
| Gallus gallus | Chicken | Gad d 5 | Preproalbumin (serum) | 615 |
| Gallus gallus | Chicken | Gad d 4 | Lysozyme C | 147 |
| Gallus gallus | Chicken | Gad d 3 | Ovatransferrin | 705 |
| Helix aspera | Snail | Hel as 1 | Tropomyosin | 284 |
| Homarus americanus | American lobster | n/a | Tropomyosin | 274 |
| Metapenaeus ensis | Shrimp | Met e 1 | Tropomyosin | 284 |
| Mimachlamys nobilis | Scallop | n/a | Tropomyosin | 284 |
| Panulirus stimpsoni | Lobster | n/a | Tropomyosin | 274 |
| Perna viridis | Mussel | n/a | Tropomyosin | 284 |
| Salmo salar | Salmon | Sal s 1 | Parvalbumin beta 1 | 109 |

The allergens listed in Table 1 are exemplary and are well known to those of skill in the art. In specific embodiments, the food allergen is one that is from a legume, such as peanut. Allergens from peanuts have been well characterized, see, e.g., U.S. Patent No. 6,486,311; U.S. Patent No. 6,441,142; U.S. Patent No. 5,558,869. In particular, the allergens Ara h1, Ara h2 and Ara h3 are known to be responsible for allergic reactions to peanuts; Ara h4, Ara h5, Ara h6, Ara h7 and Ara h8 also may be used in the arrays described herein. The sequences of each of these proteins are well known to those of skill in the art. For example, an exemplary sequence for major peanut allergen Ara h1 is found at GenBank Accession No. AAL27476 (reproduced as SEQ ID NO:1 herein); peanut allergen Ara h2 is found at GenBank Accession No. AAM78596 (reproduced as SEQ ID NO:2) another sequence for peanut allergen Ara h2 is depicted at GenBank Accession No. AAK96887 and reproduced herein as SEQ ID NO:3; the sequence of allergen Ara h3/Ara h4 is found at GenBank Accession No. AAM46958 (reproduced as SEQ ID NO:4); the protein sequence for Ara h3 (glycinin) is depicted at GenBank Accession No. AAC63045 (reproduced as SEQ ID NO:5); the sequence of peanut allergen Ara h4 (glycinin) is shown in GenBank Accession No. AAD47382 (reproduced herein as SEQ ID NO: 6); peanut allergen Ara h 5 (profilin) is found at GenBank Accession No. Q9SQI9 (reproduced as SEQ ID NO:7); another sequence for peanut allergen Ara h5 (profilin) is shown in GenBank Accession No. AAD55587 (reproduced herein as SEQ ID NO: 8); the sequence of peanut allergen Ara h6 is depicted at GenBank Accession No. AAD56337 (reproduced herein as SEQ ID NO: 9); the sequence of a conglutin-like allergen from peanut as shown is GenBank Accession No. AAD56719 (reproduced herein as SEQ ID NO: 10); peanut allergen Ara h 8 allergen is found at GenBank Accession No. AAQ91847 (reproduced as SEQ ID NO:11). Any of these sequences may be used to generate the epitope peptide arrays described herein. The sequence of peanut allergen Ara h I is shown in GenBank Accession No. AAB00861 (reproduced herein as SEQ ID NO: SEQ ID NO:12). This recombinant protein has been shown to be a peanut allergen through Ara h I expression and IgE binding studies in patients with peanut hypersensitivity (Burks et al., J. Clin. Invest. 96 (4), 1715-1721, 1995). The 614 amino acid sequence depicted at SEQ ID NO:13 (reproduced from GenBank Accession No: AAA60336) is another peanut Ara h I allergen sequence that will be particularly useful in generating the epitope peptides for the uses described herein.

In other particular embodiments, the food allergen is an allergen from cow's milk. Cow's milk allergy (CMA) is one of the earliest and most common food allergies and CMA frequently precedes other food and respiratory allergies. As such, diagnoses for CMA are particularly important in young children who may fail to thrive as a result of such allergies. Many adults also are intolerant to milk. Milk contains at least 20 protein components that may provoke an antibody response in man (Bleumink et al., Int Archs Allergy Appl Immunol; 34:521-543, 1968). Milk protein fractions are traditionally subdivided into casein and whey fractions. The caseins are generally found in micellar complexes, which give milk its "milky" appearance, and constitute from 76% to 86% of the protein in cow milk (Whitney, In: Wong NP, James R, Keeny M, Marth EH, editors. Fundamentals of Dairy Chemistry. New York: Van Nostrand Reinhold, 1988: 81-169, 1988; Swaisgood et al., In: Fennema OR, editor. Food Chemistry. New York: Marcel Dekker,: 791-827, 1985). The casein fraction is precipitated from skim milk by acid at pH 4.6 and consists of 4 basic caseins, αs1-, αs2-, β-, and κ-, which account for 32%, 10%, 28%, and 10% of the total milk protein, respectively. The primary sequences of the caseins have been established (Swaisgood et al., In: Fennema OR, editor. Food Chemistry. New York: Marcel Dekker,: 791-927, 1985), and three-dimensional structures elucidated. The non-casein fraction, or whey, accounts for ~20% of total milk protein and consists of β-lactoglobulin [9% of total milk protein], α-lactalbumin [4%], bovine immunoglobulins [2%], bovine serum albumin [1%], and minute quantities of various proteins [lactoferrin, transferrin, lipases, esterases, etc. that collectively make up 4% of total milk protein]. Several of the whey proteins are denatured by extensive heating [bovine serum albumin, bovine gamma globulin, and α-lactalbumin]. The complete primary structures of β-lactoglobulin and α-lactalbumin have been determined.

Other allergens that may be used to prepare microarrays for use in the methods described herein may include, e.g., those listed in U.S. Patent No. 5,449,669 (crustacean allergens).

It is contemplated that the methods of the present invention will be employed to determine the allergen epitope binding diversity of the IgE of a given subject. Such diversity may be assessed with respect to the epitopes from a single allergen or a panel of different allergens. Methods of producing arrays of such epitopes are described in further detail herein below.

### V. METHODS OF PREPARING PEPTIDE MICROARRAYS

As discussed herein throughout, in certain aspects of the present invention the methods of the present invention involve a microimmunoassay in which epitopes from an allergen of interest are contacted with the serum from a test subject. Preferably, the epitopes are peptide epitopes that are arranged in a microarray, the microarray also may comprise non-peptide epitopes. The microarray of peptide (or other) epitopes may be one which presents the epitopes immobilized upon a solid surface; alternatively, the epitopes may be in solution.

The use and production of microarray chips comprising nucleic acid molecules are well known to those of skill in the art (see, e.g., U.S. Patent Nos. 6,308,170; 6,183,698; 6,306,643; 6,297,018; 6,287,850; 6,291,183). Techniques used for preparing nucleic acid microarrays are applicable to the production of protein microarrays and those of skill in the art are aware of numerous other methods and compositions for producing protein-based microarrays. See in particular, MacBeath et al., Science 289(5485):1760-1763, 2000; and Shreffler et al., J Allergy Clin Immun 109[1], S286,2002.

As discussed above, protein-based microarrays provide a simple way to explore the binding of serum IgE from samples from patient sera in a diagnostic context to screen for severity of allergy to particular allergens. Such microarrays also could be used to provide a whole profile of dietary restrictions that will be helpful in planning the diet of individuals with multiple food allergies. The assays also may prove useful in prognostic applications in assisting in determining whether a given therapeutic intervention will be effective. In the present invention, at least 10 peptides having distinct sequences derived from one or more allergens may be presented in a peptide microarray for the analysis of IgE binding to specific sites on the microarray. As discussed above, preferably the peptides in the peptide array contain between 10 to about 50 amino acid residues and these sequences may be derived from any allergen(s) being tested. For example, in certain embodiments, there is provided a diagnostic array for determining the severity of an allergy to peanuts. In such an exemplary array, there are peptides derived from one or more of the sequences set forth in SEQ ID NOs:1-13.

The term "microarray" refers to an ordered arrangement of allergen epitopes. The array elements (i.e., the epitopes) are arranged so that there are preferably at least two or more different array elements, more preferably at least 100 array elements, and most preferably at least 1,000 array elements, on a 1 cm² substrate surface. The signal that will be generated from the IgE binding to any of the array elements is individually distinguishable.

The microarray can be used for large-scale IgE binding analysis of a large number of target serum samples. When the epitope peptides are arranged in a microarray on a solid substrate, the epitopes are organized on the microarray in an ordered fashion so that each epitope is present at a distinguishable, and preferably specified, location on the solid substrate of the microarray substrate. In the preferred embodiments, because the array elements are at specified locations on the substrate, the immunocomplexation of the epitopes and intensities produced from such complex formation (which together create a specific binding profile for the individual being tested) can be interpreted in terms of binding levels of particular epitopes from a given allergen and can be correlated with severity of allergic response, as discussed above. These methods will produce an IgE binding profile for the subject being tested with respect to any of the allergens to which the serum is exposed.

A composition comprising a plurality of allergen epitopes can also be used to produce an allergen profile for each individual. Even if a given epitope from a specific allergen does not bind to IgE from a subject's serum, such non-binding will be useful in that it can be determined from such non-binding that the specific epitope tested does not produce an allergic response in that individual. Typically, therefore, an allergen profile for a given subject will include epitopes which do bind to the IgE from the serum of the patient and those that do not.

### 1. Microarray Production

The peptide (or other) epitopes may be simple peptides or may be peptides linked to the solid substrate by way of an inert, non-immunological linker. In one embodiment, the epitopes are peptides of about 10 to about 25 amino acids in length; however, as explained above the size of the peptides may vary. Moreover, it should be understood that any array may comprise peptides of varying lengths, for example, one location may have a peptide A of length W, whereas another location on the array may have a peptide B of length X.

The peptides can be prepared from a variety of synthetic or enzymatic schemes, which are well known in the art. Where short peptides are desired, such peptides are prepared using automated peptide synthesis in solution or on a solid support in accordance with conventional techniques. Various automatic synthesizers are commercially available and are used in accordance with known protocols. See, for example, Stewart and Young, Solid Phase Peptide Synthesis, 2d. ed., Pierce Chemical Co., (1984);Tam et al., J. Am. Chem. Soc., 105:6442, (1983); Merrifield, Science, 232: 341-347, (1986); Barany and Merrifield, The Peptides, Gross and Meienhofer, eds, Academic Press, New York, 1-284, (1979); Fields, (1997) Solid-Phase Peptide Synthesis. Academic Press, San Diego.); Andersson et al., Large-scale synthesis of peptides. Biopolymers (Pept. Sci.), 55, 227-250 (2000); Burgess et al., DiSSiMiL: Diverse Small Size Mini-Libraries applied to simple and rapid epitope mapping of a monoclonal antibody. J. Pept. Res., 57, 68-76, (2001); Peptides for the New Millennium, Fields, J.P. Tam & G. Barany (Eds.), Kluwer Academic Publisher, Dordrecht. Numerous other documents teaching solid phase synthesis of peptides are known to those of skill in the art and may be used to synthesize epitope arrays from any allergen.

For example, the peptides are synthesized by solid-phase technology employing an exemplary peptide synthesizer such as a Model 433A from Applied Biosystems Inc. This instrument combines the FMOC chemistry with the HBTU activation to perform solid-phase peptide synthesis. Synthesis starts with the C-terminal amino acid. Amino acids are then added one at a time till the N-terminus is reached. Three steps are repeated each time an amino acid is added. Initially, there is deprotection of the N-terminal amino acid of the peptide bound to the resin. The second step involves activation and addition of the next amino acid and the third step involves deprotection of the new N-terminal amino acid. In between each step there are washing steps. This type of synthesizer is capable of monitoring the deprotection and coupling steps.

At the end of the synthesis the protected peptide and the resin are collected, the peptide is then cleaved from the resin and the side-chain protection groups are removed from the peptide. Both the cleavage and deprotection reactions are typically carried out in the presence of 90% TFA, 5% thioanisole and 2.5% ethanedithiol. After the peptide is separated from the resin, e.g., by filtration through glass wool, the peptide is precipitated in the presence of MTBE (methyl t-butyl ether). Diethyl ether is used in the case of very hydrophobic peptides. The peptide is then washed a plurality of times with MTBE in order to remove the protection groups and to neutralize any leftover acidity. The purity of the peptide is further monitored by mass spectrometry and in some case by amino acid analysis and sequencing.

The peptides also may be modified, and such modifications may be carried out on the synthesizer with minor modifications. An amide could be added at the C-terminus of the peptide. An acetyl group could be added to the N-terminus. Biotin, stearate and other modifications could also be added to the N-terminus.

The purity of any given peptide, generated through automated peptide synthesis or through recombinant methods, is typically determined using reverse-phase HPLC analysis. Chemical authenticity of each peptide is established by any method well known to those of skill in the art. In certain embodiments, the authenticity is established by mass spectrometry. Additionally, the peptides also may be quantified using amino acid analysis in which microwave hydrolyses are conducted. In one aspect, such analyses use a microwave oven such as the CEM Corporation's MDS 2000 microwave oven. The peptide (approximately 2 µg protein) is contacted with e.g., 6 N HCl (Pierce Constant Boiling e.g., about 4 ml) with approximately 0.5% (volume to volume) phenol (Mallinckrodt). Prior to the hydrolysis, the samples are alternately evacuated and flushed with N₂. The protein hydrolysis is conducted using a two-stage process. During the first stage, the peptides are subjected to a reaction temperature of about 100°C and held that temperature for 1 minute. Immediately after this step, the temperature is increased to 150°C and held at that temperature for about 25 minutes. After cooling, the samples are dried and amino acid from the hydrolysed peptides samples are derivatized using 6-aminoquinolyl-N-hydroxysuccinimidyl carbamate to yield stable ureas that fluoresce at 395 nm (Waters AccQ Tag Chemistry Package). In certain aspects, the samples are analyzed by reverse phase HPLC and quantification is achieved using an enhanced integrator.

Labels can be incorporated into the peptides by methods well known in the art. The only requirement is that the incorporated label not interfere with detectable binding of the peptide to the IgE.

The polypeptides can be immobilized onto a solid support or substrate. Preferred substrates are any suitable rigid or semi-rigid support including membranes, filters, chips, slides, wafers, fibers, magnetic or nonmagnetic beads, gels, tubing, plates, polymers, microparticles, and capillaries. The substrate can have a variety of surface forms, such as wells, trenches, pins, channels and pores, to which the epitope peptides are bound. Preferably, the substrates are optically transparent. In exemplary embodiments, the solid support is a poly-L-lysine-coated microscope slide and methods of immobilizing the peptides simply comprise application of the peptide to the solid support, as described in Example 1.

However, it is contemplated that the peptides also can be immobilized by covalent means such as by chemical bonding procedures or UV. In one such method, a peptide is bound to a glass surface which has been modified to contain epoxide or aldehyde groups. In another case, a peptide labeled with biotin may be bound to a solid support that is coated with avidin. In yet another method, a peptide is actively transported from a solution to a given position on a substrate by electrical means, as has been described for nucleic acids (Heller et al., U.S. Pat. No. 5,605,662). Alternatively, individual peptides can be gridded on a filter by UV cross-linking.

Furthermore, the peptides do not have to be directly bound to the substrate, but rather can be bound to the substrate through a linker group. The linker groups are typically about 6 to 50 atoms long to provide exposure to the attached peptide. Preferred linker groups include ethylene glycol oligomers, diamines, diacids and the like. Reactive groups on the substrate surface react with one of the terminal portions of the linker to bind the linker to the substrate. The other terminal portion of the linker is then functionalized for binding the peptide.

The peptides can be attached to a solid substrate (used herein interchangeably with the term "solid support") by dispensing reagents for peptide synthesis on the substrate surface or by dispensing preformed peptides on the substrate surface. Typical dispensers include a micropipette delivering solution to the substrate with a robotic system to control the position of the micropipette with respect to the substrate. There can be a multiplicity of dispensers so that reagents can be delivered to the reaction regions simultaneously. Exemplary peptide libraries are described e.g., in MacBeath et al., Science 289(5485):1760-1763, 2000; Shreffler et al., J Allergy Clin Immun 109[1], S286,2002.

### 2. Immunocomplexation and Detection in Microarrays

Immunocomplexation of the IgE from a test sample and the epitopes from an allergen will be detected using a variety of techniques. The serum sample is incubated with a microarray under conditions that allow immunocomplexation to take place. After this incubation period, the microarray is washed to remove excess serum that has not bound to the epitopes, and complex formation between the IgE from the serum and the epitopes on the microarray is detected. Methods for detecting complex formation are well known to those skilled in the art. In a preferred embodiment, the detection is through the use of an anti-IgE antibody that is suitably labeled.

In those embodiments where the label is a fluorescent label, measurement of levels and patterns of fluorescence indicative of complex formation may be accomplished by fluorescence microscopy, preferably confocal fluorescence microscopy. An argon ion laser excites the fluorescent label, emissions are directed to a photomultiplier, and the amount of emitted light is detected and quantified. The detected signal will be proportional to the amount of epitope/IgE complex at each position of the microarray. The fluorescence microscope can be associated with a computer-driven scanner device to generate a quantitative two-dimensional image of signal intensities. The scanned image is examined to determine the intensity of signal from each epitope.

Typically, microarray fluorescence intensities can be normalized to take into account variations in binding intensities when more than one microarray is used under similar test conditions. In a preferred embodiment, individual IgE/epitope complexation intensities are normalized using the intensities derived from internal normalization controls contained on each microarray.

In exemplary embodiments, a peptide library is synthesized. Typically, each peptide in series will contain 10-25 sequence-derived residues that overlap by some portion. In addition, two glycines and a biotin may be added to allow for site-specific immobilization and accessability for antibody binding. The peptides are synthesized by solid phase f-moc chemistry in, e.g., 96 parallel-synthesis sets (PepSet, Mimotope). This yields micromole quantities of each individual peptide. The peptides are immobilized onto avidin-functionalized glass microscope slides (Corning), rinsed with PBS and stored until use. In one exemplary assay, 60 µl of diluted (1:5 for IgE detection using PBS with 0.5% tween 20 and 1 % BSA [PBS-T-BSA]) patient or control serum is be incubated for 1 hour under a 22 mm² coverslip. Following rinsing with x5 with PBS-T, bound human IgE will be detected with goat anti-human IgE (1:1000 in 80 µl PBS-T-BSA for 30 min; Molecular Probes). Slides are rinsed in PBS-T followed by water and centrifuged to complete dryness. Fluorescence is then measured at 670 nm using a GeneArray Reader (Affymetrix, CA).

In another exemplary assay, 80 µl of human serum diluted 1:5 with PBS-T-BSA is incubated over the microarray at room temperature in a humidified chamber. The slides are rinsed five times with PBS-T and subsequently incubated with a cocktail of anti-human IgE-Alexa 647 and anti-human IgG4-Alexa 546 for two hours at room temperature. The slides are then rinsed in PBS-T, dried and fluorescence is measured using a GeneArray Reader (Affymetrix, CA).

Image analysis, data capture and analysis is performed using ScanAlyze (Michael Eisen, Stanford University) and a VBA/ Microsoft Access application. Each array contain whole recombinant or purified control proteins used for quality control and normalization (Poirier et al., Allergy Asthma Proc. 18(6):359-362, 1997). Significance of antibody binding at each peptide is determined by statistical comparison of binding by a negative reference population. Comparison of different patient or control groups with respect to specific epitopes recognized, as well as antibody diversity (i.e. number of epitopes recognized), is the performed. Digitized fluorescence values for each element on a slide were log transformed and normalized by comparison to a positive control present on each slide.

### 3. Allergenicity Profiles Generated from Microarray

Allergenicity profiles may be prepared using the methods of the present invention. An allergenicity profile can be used to assess those allergens to which the subject is allergic and those to which it is tolerant; thereby aiding in modifications to diet and/or lifestyle to improve the quality of life of the individual.

The allergenicity profile includes a plurality of detectable complexes formed between the IgE of the individual and a plurality of epitopes from a one or more allergens. Each complex is formed by binding of serum IgE from the subject to one or more epitopes of one or more allergens. At least one of the epitopes from at least one of the allergens, and preferably a plurality of epitopes from a plurality of allergens, will produce a profile of the moieties to which a given subject is allergic. A complex between the epitope and the IgE from the serum of the subject is detected as described above. The allergenicity profiles provide "snapshots" that can show unique allergic responses that are characteristic to that specific individual.

After performing the immunocomplexation experiments between the serum IgE and the epitopes on the microarray and interpreting detected signals from the microarray, particular epitopes are identified and selected as those to which the individual is particularly susceptible. Such epitope sequences can be used to ascertain specific medicaments and or dietary/environmental changes or restrictions for the particular individual.

A plurality of epitopes can be used as elements in a microarray. Such a microarray can be employed in several applications including diagnostics, prognostics and treatment regimens, drug discovery and development, toxicological and carcinogenicity studies, forensics, pharmacogenomics, and the like for the identification and/or treatment of allergies and anaphylaxis potential.

In one situation, the microarray is used to monitor the progression of an allergic response in response to a given medicament. Researchers can assess and catalog the differences in IgE binding to epitopes before and after treatment. By analyzing changes in patterns of IgE binding to the epitopes, the efficacy of the treatment can be monitored. Also, researchers can use the microarrays to rapidly screen large numbers of a population to determine the effects of a given allergen on a population.

### VI. FUNCTIONAL CHARACTERIZATION OF IgE

In some embodiments, it may be desirable to further characterize and assess the IgE response of a given patient. In such embodiments, the subject's humoral immune response is measured by assessing the functional capacity of subjects' IgE for activating allergen-stimulated, passively sensitized RBL-SX38 cells (Wiegand et al., J Immunol., 157(1):221-230, 1996). For such an assay, cells are harvested and incubated in 15 ml conical tubes with 5% patient serum in DMEM for 60 min at 37°C. The cells are then washed in PBS-2mM EDTA, resuspended in DMEM and distributed in 96 well U-bottom plates. Antigen, anti-IgE, or a mock protein is added to the wells in triplicate and incubated for 20 min at 37°C. The reaction is then stopped by addition of 1/10th volume of PBS-20 mM EDTA and centrifuged at 300 g. Supernatants from the wells are collected and stored at -70 °C until measurement of histamine can be taken. Histamine is measured by automated fluorometric assay.

In another assay, it may be desirable to monitor the T-cell and basophil responses in subjects that are identified as having an allergic reaction to a given allergen. In such embodiments, the phenotype and cytokine profiles of the T-cells of the subject are assessed. In order to do this, the subject's lymphocytes are isolated and cultured. Lymphocyte isolation and culture techniques are well known to those of skill in the art. In an exemplary protocol, PBMCs are isolated by Ficoll-Hypaque density gradient centrifugation and then 5 x10⁶ cells are resuspended in a total volume of 5 ml of 5µM carboxy fluorescein succinimidyl ester (CFSE) and incubated for 15 min at 37°C in the dark. CFSE has been used extensively to label living cells and follow their proliferation in vivo and in vitro over time (days) by flow cytometry. The stain does not transfer between cells and is equally partitioned between daughter cells following each cell division, allowing for measurement of proliferation and calculation of percent responding cells (Lyons, J Immunol Methods, 243(1-2):147-154, 2000). Cells are cultured for 5 days in either medium alone, with 250 µg/ml allergen to be tested, or 1 µg/ ml Staphylococcus enterotoxin B (SEB). Culture medium may be supplemented with glutamine and streptomycin.

To analyze the cytokine profiles of the T-cells, flow cytometry may be used. Cells are stimulated with PMA (5 ng/ ml), calcium ionophore (250 ng/ml) (Sigma) in the presence of a protein transport inhibitor brefeldin A (10µg/ ml) (BD Pharmingen) for four hours at 37°C. Cells are then harvested, washed and incubated with fluorochrome-labeled anti-CD45RO, -CD11a, -CD95 and adequate isotype controls. Cells are fixed and permeabilized (BD Pharmingen) and then stained with anti-CD4, -CD8, -IL4, -IL5, -IL13, -IL2, -IFNγ and adequate isotype controls. Fluorescence data is acquired in five-color mode on a FACS Vantage (BD Biosciences). At least 2 x 10⁴ light-gated events are acquired for each condition. Software analysis and additional offline compensation as necessary may be performed using FlowJo (TreeStar). Antigen-specific proliferation is determined by a comparison of CFSE low events with antigen stimulation versus those with medium alone. The phenotype of proliferating cells with respect to cytokine and surface markers can then be compared to resting cells as well as SEB-responding cells.

In some embodiments, the diagnostic assays may be supplemented with experiments to assess-the effects of allergens on T-cell lines from mucosal and peripheral blood lymphocytes. Such assays are useful in demonstrating that T-cell response to an antigen is present locally (e.g., in the gut) and in the circulating lymphocytes. In such assays, biopsies from the duodenum in subjects undergoing scheduled biopsies for diagnostic purposes are placed into sterile collection medium (calcium- and magnesium-free Hank's balanced salt solution with 5% AB-serum and penicillin plus streptomycin). The specimens are washed three times with collection medium and twice with culture medium, minced into small fragments and placed in 24 well plates in culture media containing rIL-2 (20 IU/ml, Sigma) and allergen to be tested (50 µg/ml each) (37°C, 5% CO2). Cells are examined daily and the media exchanged as necessary.

In parallel, allergen-specific T-cell lines from the peripheral blood also may be established. Peripheral blood mononuclear cells (PBMCs) are isolated by Ficoll-Hypaque density centrifugation techniques known to those of skill in the art. Similar to the gut biopsies PBMCs are stimulated for e.g., 14 days with allergen of choice, e.g., at a concentration of 50µg/ml in the presence of IL-2. Cultured PBMCs and mucosal cells are adjusted to 2x10⁷ cells/ml and T-lymphocytes are purified using magnetic beads coated with anti-CD2 mAb (Dynal). The remaining cells including monocytes and/or dendritic cells as possible antigen presenting cells (APCs) are centrifuged at 1500 rpm and re-suspended in 1 ml of culture medium. Purified T cells are then detached from the magnetic beads, re-suspended in culture medium and labeled with CFSE as described above.

Both mucosal and peripheral T-cells are cultured with APCs from the blood and gut (typically, APCs from the gut remain functional in culture for about 30 days). Therefore, 500 µl of culture medium containing 5x10⁶ purified, detached and CFSE-labeled mucosal T-lymphocytes are added to 500 µl of culture medium containing APCs from the gut or peripheral blood. Purified, detached and CFSE-labeled lymphocytes from the peripheral blood are similarly treated. Cells are cultured for 5 days in the presence of, e.g., 200 µg/ml allergen proteins. After 3 days, 500 µl of supernatant is removed for cytokine measurements and replaced with fresh culture medium containing 20 IU/ml rIL-2. Cell culture supernatants are harvested after 3 days of co-culture experiments [and replaced with free fresh culture medium containing 20 IU/ml rIL-2], aliquoted and frozen at -80°C until further analysis. ELISA for IL-4, IL-5, IL-13, IL-10, TGF-β and IFN-γ are performed following the instructions of the manufacture [Pharmingen, San Diego, CA]. Lymphocyte proliferation and intracellular cytokine production also may be assessed as described above.

Histamine release by peripheral blood basophils also may be assessed to determine the response of the individual to a given allergen. In such determinations, PBMCs are isolated, e.g., using Ficoll separation, from subjects that have been identified using the methods of the invention as having an IgE-mediated allergy. The PBMCs are stimulated with various concentrations of allergen proteins [e.g., 20 ng/ml to 20 µg/ml], anti-IgE and ionophore as positive controls, or medium alone for 6 hours with rIL-3 and the protein transport inhibitor, Brefaldin A. Cells are then labeled with fluorochrome-tagged antibodies against CD123, CD203c, HLADR and CD63, fixed and permeablized, stained for intracellular IL-4 and IL-13, and evaluated by flow cytometry. The supernatants can be collected for histamine measurements using automated fluorometric assay.

The above assays are merely exemplary assays that may be used to supplement the diagnostic methods of the present invention to produce more detailed characterizations of the subject's response to a given allergen. However, it should be understood that the microimmunoassay alone will be sufficient to provide a diagnostic evaluation of the degree of severity of an immune response a subject will exhibit in response to any given allergen.

### VII. ANTI-IGE TREATMENTS AND METHODS OF PREDICATING EFFICACY OF SAME

Anti-IgE-based therapies are now becoming a popular line of attack against allergic disorders including asthma and various other IgE-based allergic responses. Anti-IgE antibodies are well known to those of skill in the art and act by attaching to IgE in the blood and other tissues, and thereby preventing or otherwise diminishing the release of histamine and decreasing the severity of the IgE allergic reaction. Anti-IgE compositions are well know to those of skill in the art, see e.g., U.S. Patent No. 6,761,889 and U.S. Patent No. 6,682,735 (directed to anti-IgE antibodies); U.S. Patent No. 6,723,833 (directed to therapeutic compositions comprising anti-IGE antibodies and immunosuppressive agent; U.S. Patent No. 6,579,688 (directed to stabilizing diluents for polypeptides and antigens); U.S. Patent No. 6,504,013 directed to canine allergy therapeutic recombinant anti-IgE monoclonal antibody compositions); U.S. Patent No. 6,290,957 and U.S. Patent No. 5,994,511 each directed to Anti-IgE antibodies and method of improving polypeptides; U.S. Patent No. 5,543,144 directed to treatment of hypersensitivities with anti-IgE antibodies that are specific for IgE-expressing B cells but not basophils; U.S. Patent No. 5,449,760 directed to monoclonal antibodies that bind soluble IgE but not IgE on B-lymphocytes or basophils; U.S. Patent No. 5,428,133 chimeric anti-human IgE monoclonal antibodies which bind secreted IgE and membrane-bound IgE on B cells but not IgE bound to FC receptors on basophils; U.S. Patent No. 5,422,258 directed to methods of producing high affinity anti-human IgE monoclonal antibodies which bind to IgE on B cells but not on basophils; and U.S. Patent No. 5,420,251 directed to anti-idiotype antibodies for the aratope of antibodies which bind IgE bearing B cells but not basophils. Any of the antibody compositions or techniques for making the anti IgE compositions described in the aforementioned patents may be used to generate anti-IgE antibodies for use in the methods of the present invention. Commercially available anti-IgE antibody compositions also are available. An exemplary such composition is Omalizumab (Xolair®, Genentech, Inc., San Francisco, Ca), which is thought to work by binding free IgE in the serum and reducing the capacity of such IgE to bind to the Fc epsilon RI.

While it is recognized that anti-IgE treatments will be useful for the treatment of allergies such as asthma, peanut allergy, allergic rhinitis, atopic dermatitis and the like, it has been noted by the present inventors that there is significant variation in the responsiveness of patients to a given anti-IgE dose. While many patients known to have a specific allergy, when treated with anti-IgE antibody were more tolerant of those allergens as compared to a group of patients having a similar allergy but not being treated with anti-IgE, it was found that there were certain patient populations who were non-responsive, or less responsive than expected, to anti-IgE therapy. It is suggested that these patients are refractory to anti-IgE treatment due to the fact that these patients have a greater allergenic epitope diversity.

The degree of heterogeneity among sensitized individuals with respect to their epitope binding patterns as determined using the diagnostic methods described herein will be used to assess whether a patient will be responsive to a given anti-IgE antibody-based therapy. As noted above, greater diversity of peptide epitope recognition correlates only weakly with allergen-specific IgE levels (i.e., amount of KU/L), however, these patients while manifesting a more severe allergic response are not readily treatable with typical and low doses of anti-IgE therapy. As such these patients may be unresponsive or less responsive to standard treatment with such anti-IgE antibody compositions. In the present invention, however, patients with IgE binding to a greater number of epitopes are identified as those individuals that will benefit from an increased dose of anti-IgE therapy, or who may not be able to be adequately protected. The present invention therefore identifies patients who will be responsive to anti-IgE therapy by determining the allergenic epitope diversity of the patients as described herein. Such methods allow identification and treatment of patients that manifest severe allergies that were previously thought to be non-treatable with or non-responsive to anti-IgE antibodies such as Xolair®.

### VIII. EXAMPLES

The following examples are included to demonstrated preferred embodiments of the invention.

### EXAMPLE 1

### MATERIALS AND METHODS

The present example provides exemplary methods and materials used in the preparation and testing of a microimmunoassay for determining the severity of an allergic response.

***Collection and Processing of Patient Sera**.* Patient and control sera were selected retrospectively on the basis of detectable IgE to peanut as measured by Pharmacia Cap System FEIA. Patients that exhibited some history of food allergenicity were recruited and detailed food reaction histories were prepared. Medical records were reviewed for history of reaction frequency and symptoms. Reaction severity was ranked on a scale of 0 to 6 as described previously (Sampson, Pediatrics, 111:1601-8, 2003). The age of peanut sensitized individuals at the time of recruitment ranged from 1.5 to 36 years (median 6.9 yrs, n=57). Peanut-specific IgE ranged from 1.97 to >100 kU/L (median >100). Of these patients, 38 had a history of positive DBPCFC or convincing history of clinical reaction. The remaining 18 had elevated IgE levels predictive of clinical reactivity (Sampson, J. Allergy Clin. Immunol., 107:891-6,2001) (>15 kUA/L, median >100) but with no history of known ingestion. Additional sera from patients with confirmed clinical peanut allergy (n=20) were obtained and have been previously described (Burks et al., Eur. J. Biochem., 245:334-9, 1997). Control sera were from non-peanut allergic patients (n=10) or from healthy volunteers (n=5).

***Preparation of Microarray.*** A set of 20 amino acid peptides [20-mers] with 15 amino acid overlap were commercially synthesized and stored at 10 mg/ml PBS at -70 °C. These peptides were diluted to 0.3 mg/ml in PBS/ 30% glycerol and spotted in triplicate on poly-L-lysine coated microscope slides (Electron Microscopy Sciences, Ft. Washington, PA) 375 nm apart using a four-pin GMS 417 arrayer (Affymetrix, CA). Two peptides derived from Ara h 3 sequences, amino acids 146-165, and 151-175 (sequence from Rabjohn, 1999), were excluded from printing because they were insoluble. Four grids were created within a less than 22 x 22 mm² area. These were allowed to air dry and stored in a desiccator at room temperature until used.

***Immunoassay.*** All incubations were carried out under a standard 22 x 22 mm² coverslip (Corning) in a humidity chamber (The Binding Site). An area just larger than a coverslip around the array was outlined on all four sides with a PAP pen. Slides were pre-incubated with 80 µl PBS-T (PBS with 0.1% Tween 20) containing 1% HSA (PBS-T-HSA) for 60 minutes at 23°C. The slides were rinsed 5X in PBS-T and 80 µl of human serum diluted 1:5 in PBS-T-HSA was incubated for 30 minutes at 23°C. Following rinsing with PBS-T, 80 µl of polyclonal rabbit anti-human IgE (DAKO) diluted 1:500 in PBS-T-HSA was incubated for 30 minutes at 23 °C. Slides were then rinsed five times with PBS-T and incubated with 150 µl biotinylated anti-rabbit IgG (Link, DAKO). Slides are again rinsed and incubated with 100 µl Avidin Cy3 (Amersham) diluted 1:1000 in PBS-T for 30 minutes at 23 °C. Slides were finally rinsed with PBS-T 5x, then dH₂O and centrifuged to dryness (1000 x g in empty 50 ml conical tubes). Fluorescence emission was measured at 550 nm using GMS 418 Array Scanner (Affymetrix, CA). One peptide from Ara h 2, residues 90-109, was excluded from analysis due to non-specific binding of the secondary antibody.

***Analysis.*** Fluorescence intensity was digitized using ScanAlyze (M. Eisen, http://rana.lbl.gov/EisenSoftware.htm) and exported as tab-delimited text. Raw data files were imported into a MS Access/ VBA (Microsoft Corporation, Renton, WA) application to relate the data to the appropriate peptide identity. Data were then exported to MS Excel (Microsoft) and the mean intensity of intra-assay triplicates for each peptide was plotted against its CV to identify poor quality data, which were excluded. Each slide included negative (human serum albumin) and positive (a non-specific biotinylated protein) controls. Relative signal from each peptide was defined as the mean replicate value divided by the positive control mean replicate value.

For inter-assay comparison, the intra-assay values were log₁₀ transformed for normalization and improvement of variance homogeneity and then adjusted to set the median from each array equal to zero. In order to define positive levels of inter-assay IgE binding, a set of samples from fifteen non-peanut allergic donors including non-atopic (n=5) and atopic (n=10) individuals were selected. The inter-assay peptide cutoff was defined as the mean plus two standard deviations (SDs) of signal from this control group. For the purpose of this study, the cutoff for positive binding was set at the larger of either the inter-assay individual peptide cutoff or the intra-assay cutoff (mean plus two SD for all peptides, usually about 0.2).

***Basophil assay.*** Basophils were enriched together with PBMC from normal healthy donors by Ficoll separation of blood diluted 1:1 with PBS. Washed cells were incubated overnight in complete medium with or without patient serum containing peanut-specific IgE. Cells were then washed twice and 100 µl/well of cell suspension at a concentration of 10⁶/ml were distributed to 96-well flat-bottom tissue culture plates in serum free medium containing IL-3 (2 ng/ml, R&D Systems, Inc., Minneapolis, MN). Antigens were serially diluted at 2X final concentrations in AIM-V plus IL-3 (2 ng/ml) and 100 µl were added to the wells and allowed to incubate at 37°C and 5% CO₂ for 30 minutes. Negative and positive control wells contained medium alone or anti-IgE (0.5 µg/ml), respectively. Degranulation was measured by flow cytometry as the percent CD203c positive cells expressing high levels of CD6311, (Hauswirth, J Allergy Clin Immunol., 110:102-9,2002). Briefly, following activation, cells were harvested and washed in PBS containing 2 mM EDTA and 1% BSA (PBS-EDTA-BSA). Cells were then resuspended (approx. 8 x 10⁴) in 50 µl of PBS-EDTA-BSA and stained with previously titrated amounts of anti-CD63 FITC (clone H5C6, BD, San Jose, CA), and anti-CD203c PE (clone 97A6, Beckman Coulter, Palatine, IL). Unstained, compensation, and isotype controls were also prepared from the same donor cells. At least 50,000 events for each sample were acquired on a FACSCalibur (BD, San Jose, CA) and data were compensated and analyzed using FlowJo (Tree Star).

***RBL assay.*** The human FcεRI-transfected rat basophilic leukemia cell line, RBL-SX38, was cultured in the presence of 1.0 mg/ml G418 (Invitrogen) as previously described (Wiegand et al., J. Immunol., 157:221-30, 1996). Before reaching confluence, cells were harvested, washed and counted. In a total of 100 µl complete medium, 10⁶ cells were sensitized with saturating concentrations of human serum for 1 hour at 37°C. These cells were then washed and diluted to 10⁴/ml and from this, 200 µl was distributed per well to 96-well flat-bottom tissue culture plates. After incubating for 2 hours at 37°C to allow cells to adhere, the medium was replaced with EMEM containing serially diluted antigen. Control wells were replaced with EMEM alone or containing anti-IgE (0.5 µg/ml). Cells were incubated for 30 minutes at 37°C. Supernatants were analyzed for histamine content by an automated fluorescence-based assay (Astoria™ Micro Flow Analyzer, Astoria Pacific International, Clackamas, OR).

***Statistics.*** Patient vs. control IgE mean binding to recombinant allergens were compared by unpaired t-test, and p values reported are two-tailed. The Mann Whitney U test was used to analyze differences in epitope diversity between patient groups.

### EXAMPLE 2

### ASSAY VALIDATION

In order to assess assay characteristics, replicate experiments were performed using a pool of peanut-allergic patient sera to investigate inter- and intra-assay reproducibility. Each array also included whole recombinant Ara h1, Ara h2 and Ara h3. The same pooled sera were applied to four separate arrays. Intra-assay coefficient of correlation for any one of the four assays was >0.95 and the variance was homogenous. The inter-assay mean coefficient of variation for peptide binding was 3.4% with a range of 0.1% to 14%, which correlated with intensity of staining.

Ara h1, Ara h2 and Ara h3 have been previously described as major peanut allergens (Rabjohn et al., J. Clin. Invest., 103:535-4210, 1999; Burks et al., J. Allergy Clin. Immunol. 88(2):172-9, 1991; Burks et al., J. Allergy Clin. Immunol., 90:962-9, 1992). The MIA described herein allowed examination of the prevalence of IgE recognition of these allergens for a large number of patients (n=77). Consistent with previous results, the majority of peanut-sensitized patient sera contained IgE to these recombinant proteins (Table 2). 97% of these patient sera contained specific IgE to at least one of the three allergens, while 77%, 75% and 77% recognized Ara h1, 2 and 3, respectively.

**Table 2. Patient IgE recognition of recombinant peanut allergens by MIA**

| Allergen | binding pattern | | | | | | | Percent positive^{a} |
|---|---|---|---|---|---|---|---|---|
| Arah1 | X | X | | X | X | | | 77 |
| Arah2 | X | | X | X | | X | | 75 |
| Arah3 | X | X | X | | | | X | 77 |
| Percent positive^{a} | 50.6 | 10.4 | 10.4 | 9.1 | 6.5 | 5.2 | 5.2 | 97.4^{b} |

| | | | | | | | | |
|---|---|---|---|---|---|---|---|---|
| ^{a}n=77 ^{b}Sum of row, i.e., percent positive to at least one protein | | | | | | | | |

The comparison of binding between patients and controls is shown in Figure 1. The mean signal ratio for Ara h1 was 0.72 for patients vs. 0.14 for non-peanut allergic control serum (95% confidence range, 0.38 - 0.78; p< 0.0001). For Ara h2 and Ara h3 the respective means were 0.77 (0.42-0.88) and 0.86 (0.64-0.90; p< 0.0001 for each), respectively. There was no significant difference when individuals with confirmed clinical peanut allergy were compared to those with predictive peanut-specific IgE levels, but unproven clinical status.

Figure 2A is a bar graph showing the frequency of patient serum samples with positive IgE binding to each peptide displayed on this array for 77 patients. Peptides are ordered on the x-axis from N- to C-termini of each of the three allergens (Ara h 1, Ara h 2 and Ara h 3). The dark bars represent location of the epitopes previously defined by SPOTS membrane mapping (Rabjohn et al., J. Clin. Invest., 103:535-42, 1999; Shin et al., J. Biol. Chem., 273:13753-9, 1998; Stanley et al., Arch. Biochem. Biophys., 342:244-53, 1997). Thirty-nine peptides, defining 21 major antigenic regions, are recognized by more than five patients. Ara h1 contains thirteen prominent antigenic regions, while Ara h2 has four and N-Ara h3 also has four. No single peptide was recognized by more than 35% of the samples tested. However, 67/77 (87%) of these samples were positive for at least one peptide. The mean number of peptides recognized was 9.4 and the median was 5, with a range of 0 to 56.

As can be seen, there is substantial agreement between the two methods in the identification of IgE-binding regions. The use of 20-mers produces a 'lower resolution' map; however, all of the regions of Ara h1 and Ara h2 previously identified are recognized using the microarray immunoassay as well. Quite unexpectedly, this was not the case for Ara h 3, for which very few individual sera of these patients recognized the first previously identified epitope. In addition, no single epitope is recognized by more than 35% of the patients on this platform - a striking difference from what has been published using SPOTS analysis, which identified single epitopes recognized by the majority of individual samples assayed, albeit with significantly fewer patients analyzed (Beyer et al., J Allergy Clin Immunol., 112:202-7, 2003; Rabjohn et al., J. Clin. Investigation 103:535-42, 1999; Shin et al., J. Biol. Chem., 273:13753-9, 1998; Stanley et al., Arch. Biochem. Biophys., 342:244-53, 1997).

One region of Ara h1, represented by peptides 73 and 74, and corresponding to residues 361-385, showed significant IgE binding using the microarray assay. Identification of this epitope suggested that this region may have been missed in previous analyses by virtue of the fact that initial mapping was carried out using pooled sera. This region was reanalyzed by SPOTS membrane immunoassay using these and other individual sera identified as recognizing peptides 73-74. These sera also recognized this region by SPOTS analysis, confirming the result obtained by MIA and underscoring the advantages of using individual sera for epitope mapping.

### EXAMPLE 3

### DIAGNOSTIC FINDINGS

A striking feature of IgE binding revealed by MIA is the variability from patient-to-patient with respect to both the number of peptide epitopes recognized and the pattern of recognition. Figure 2B shows eight representative graphs, plotting signal against peptide, that demonstrate this large degree of heterogeneity. These data demonstrate that the MIA methods used herein can be used to produce a map of an individual subject's heterogeneous patterns of IgE binding to various allergens. Such mapping will assist in therapeutic and prophylactic intervention of allergic responses of the subjects. In particular, the methods described herein demonstrate that greater IgE diversity with respect to epitope recognition corresponds to a history of greater reaction severity such that greater epitope diversity is a marker for more severe allergic reactions. Food antigen-specific IgE concentrations alone have been shown to have prognostic value for likelihood of reactivity (Sampson, J. Allergy Clin. Immunol., 107:891-6, 2001; Boyano-Martinez et al., J. Allergy Clin. Immunol., 110:304-9, 2002). For the patients in this study, who as a group have high peanut-specific IgE levels (median >100 kU/L), there was no significant correlation between IgE and historical reactivity.

For assessment of IgE diversity, patients were divided into those with cutaneous symptoms alone (n=14) versus those with multiple organ system involvement (n=24). The patients with a history of more severe, multi-system reactions recognized a significantly greater number of epitopes than those with a history of cutaneous symptoms alone (median 9.5 vs. 2, respectively; p<0.01; Figure 3A). The same patients have similar recognition of whole recombinant peanut allergens (Figure 3B).

In addition, when all peanut-sensitized patients were segregated by median number of positive epitopes recognized (5 epitopes), those with greater epitope diversity had a history of significantly more severe reactions. For the low diversity group, the 95% confidence interval for reaction score was 0.67 to 1.5, while for the high diversity group, the interval was 1.3 to 2.2 (p<0.05, n=27 for each group). This is not the case when these patients were segregated by quantity of recombinant peanut protein-specific antibody or CAP-FEIA peanut-specific IgE.

A related trend was seen with respect to the number of documented reactions. The high diversity group had a median of 1.5 reactions (range 0 to 6) versus the low diversity group with a median of 0 reactions (range 0 to 3, p=0.08). In comparison, when segregated by median recombinant peanut allergen binding, both groups had a similar number of reactions (median = 1; p=0.96).

To test the correlation of patients' histories of reactions to epitope diversity recognition, two bioassays of IgE function were adapted. In the first set of experiments, basophils from non-peanut allergic donors were incubated with pooled sera from individuals with high or low diversity peanut-specific IgE (n=4; median IgE > 100 kU/L for each group). These pooled sera were matched for ratio of peanut-specific to total IgE and used at equal concentrations. Following sensitization, cells were stimulated with peanut extract and degranulation was measured as increased surface expression of CD63 20-23. Figure 4A-C shows representative results from one passively sensitized normal non-atopic donor (Figure 4A) and one atopic, non-peanut allergic donor (Figure 4B). In both cases, the high diversity IgE sera conferred significantly greater sensitivity to peanut extract (p<0.001, p<0.05, respectively). The greater response was specific to peanut allergen; no difference in the response to anti-IgE was seen for either donor. To further test the functional capacity of these two pooled sera, the rat basophilic leukemia cell line expressing human FcεRI (RBL-SX38) was used (Wiegand et al., J Immunol., 157(1):221-230, 1996; Dibbern et al., J. Immunol. Methods, 274:37-45, 2003). Consistent with the results with passively sensitized human basophils, the more diverse IgE-containing sera conferred greater effector response (Figure 4C, p < 0.001).

In view of the above results, it has been demonstrated herein that epitope diversity correlates with severity of allergic response. As such, the microarray-based analyses described herein for peanut allergens may readily be adapted for any allergen to diagnose the severity of response such an allergen(s) may elicit. As demonstrated above, individual allergenic responses to any given allergen varies from individual to individual. The methods of the present invention can be used to prepare specific profiles of allergens to which any given individual is allergic. Such profiling of epitope diversity and its progression or regulation should lead to improved therapeutic strategies and better monitoring of immunotherapeutic interventions. Surveillance addressing the phenomenon of determinant spreading and its potential relationship to allergy persistence or tolerance is also amendable to the methods of the invention. The microarray immunoassays described herein makes such large-scale epitope surveillance studies practical.

### EXAMPLE 4

### METHOD OF DETERMINING RESPONSE TO OMALIZUMAB THERAPY

In assessing the responsiveness of patients to anti-IgE treatment, it was found that there was a significant variation in response of patients to the 300 mg and 450 mg doses of anti-IgE antibody [**TNX-901**] [Leung, Sampson, et al. NEJM 2003]. Generally, the patients treated with anti-IgE antibody tolerated significantly more peanut protein [2.8 gm] as compared to placebo-treated patients [~0.9 gm]. However, about 25% of the patients that were treated with anti-IgE tolerated 8 gm of peanut protein while another 25% tolerated no more peanut protein than they did prior to the treatment with the anti-IgE, indicating that there is a significant population of patients that appear non-responsive to anti-IgE therapy. This lack of responsiveness to anti-IgE therapy did not appear to correlate with total serum IgE or peanut-specific IgE levels.

The data presented in Examples 1-3 above show that there is a correlation between allergenic epitope diversity, i.e. number of allergenic epitopes recognized by an individual [as defined by peptide microarray assay methods described herein], and the severity of allergic reaction that patients experience and the amount of allergen necessary to "activate" their mast cells and basophils. In the present example it is suggested that allergic patients (e.g., patients with peanut allergies) with greater epitope diversity will receive less protection from a given dose of anti-IgE as compared to patients with lesser epitope diversity. In the present example, it is proposed that all patients are evaluated for IgE and IgG4 epitope binding to a plurality of epitopes. For the peanut allergic individuals, all patients should be evaluated for IgE and IgG4 epitope binding to Ara h1, h2 and h3 using the peptide microarray assays described herein. The following method provides an exemplary teaching of the microarray methods to be used.

**Microarray.** Lyophilized peptides are dissolved in 40% acetonitrile, 20% DMSO to 1.6M, followed by dilution in Protein Printing Buffer (ArrayIt) to a final concentration of 100 µM and stored in 384 well polypropylene plates (Matrix Technology). Triplicates are spotted on SuperEpoxy® Substrates (ArrayIt) 350nm apart using a GMS Arrayer (Affymetrix). Peptides are allowed to bind via free amines to the slide. 16 grids of 144 spots each are created within a 25 by 25mm² space on the slide. Protein Printing Buffer alone spots serve to define the background of the slide. Recombinant Ara h 2, purified in our laboratory as described elsewhere serve as positive control. The slides are air-dried in a horizontal position and stored in a desiccator until used.

**Immunolabelling.** An area around the printed grids is encircled with a PAP Pen. Slides are rinsed with PBS 0.5% Tween 20 (PBS-T) to decrease the hydrophobicity of the surface. After removing the PBS-T by suction, slides are blocked with 200 µl PBS-T with 1% Human Serum Albumin (PBS-T/HSA) for 60 minutes on a rotating platform (Labline) at ambient temperature. After removing the PBS-T/HSA from the slide surface, 200µl of serum, diluted 1:5 in PBS-T/HSA is applied to the encircled area on the slide. Slides were incubated overnight on a rotator at ambient temperature. Slides are washed and then incubated for 150 minutes with 200 µl mouse monoclonal anti-human IgE (Pharmigen) labeled with AlexaFluor® 647 (Molecular Probes) and mouse monoclonal anti-human IgG₄ tagged with AlexaFluor® 546 in PBS-T/HSA at a concentration of 0.14 (mg/mL at room temperature in the dark. All incubations are performed in a humidity chamber (The Binding Site). Subsequently, the slides are washed 3 times for 3 minutes in PBS-T in a washing station (ArrayIt), followed by 3 times rinsing in dH₂O and centrifuged to dryness in empty 50mL conical tubes at 200g for 12 minutes. Fluorescence emission is measured using a GMS 418 Array Scanner (Affymetrix) and saved as a TIF.file.

**Analysis.** Fluorescence intensity is digitized using ScanAlyze Software (Michael Eisen, Lawrence Berkeley National Lab (LBNL) and the University of California at Berkeley (UCB)) and exported as a DAT.file. A Microsoft Excel Visual Basic Application created by our laboratory is used to relate the grid data to the corresponding peptide identity and to calculate the spot intensity above the background of the slides, the average and standard deviation of the triplicates. The average plus 2 SD log intensity of the Protein Printing Buffer Spots was used as an intra-slide cutoff to establish background (1000 digital fluorescence units). Array elements with CV >30% are excluded from analysis of percent above intra-slide cutoff. An inter-slide cutoff is calculated as the average plus 2 SD intensity of the corresponding signal from the non-atopic control sera. For calculation of percent of patients with positive IgE binding, both intra- and inter-slide cutoffs must be exceeded. For hierarchical cluster analysis, IgE/ IgG₄ ratio of log-transformed data from all 55 patients and controls and all array elements are used.

By performing the above experiments it can be determined whether a patient having high epitope diversity would benefit from a higher dose of anti-IgE therapy. In some patients it may be determined that insufficient anti-IgE can be given to provide meaningful protection, thereby eliminating the need for a costly intervention that would provide no clinical benefit. On the other hand, individuals with low epitope diversity could be assured of more complete protection from the use of anti-IgE therapy.

## Claims

1. A method of predicting the severity of an immunological response against an allergen in a subject, wherein a mild immunological response involves cutaneous symptoms and a severe immunological response involves multiple organ systems, the method comprising binding IgE from the serum of said subject to a plurality of epitopes from an allergen and determining the degree of epitope binding diversity of the IgE from the serum of said subject to a plurality of epitopes on said allergen, wherein IgE binding to four or more epitopes on said allergen indicates a severe allergic response, wherein IgE binding to less than four epitopes indicates a mild allergic response, and wherein the allergen is a food allergen responsible for IgE-mediated hypersensitivity.

2. The method of claim 1, wherein the epitopes from said allergen are arrayed on a solid support.

3. The method of claim 1, wherein said binding of said IgE from the serum of said subject is carried out on a microarray immunoassay.

4. The method of claim 1, wherein said allergen is a protein.

5. The method of claim 3, wherein the epitopes from the protein are arrayed on a solid support as a series of overlapping peptide fragments of said allergen.

6. The method of claim 5, wherein said IgE from the serum of said subject binds to sequential epitopes on said allergen.

7. The method of claim 1, wherein said food allergen is an allergen from a foodstuff selected from the group consisting of a legume, a tree nut, a cereal grain, a fruit, a vegetable, a seed, a fish, a crustacean, a mollusc, poultry and a dairy product.

8. The method of claim 7, wherein said allergen is a peanut allergen selected from the group consisting of Ara h1, Ara h2 and Ara h3.

9. The method of claim 8, wherein said binding of said IgE from the serum of said subject to said peanut allergen is carried out using a microarray immunoassay, wherein said microarray comprises an array of overlapping peptides from one or more of the allergens Ara h1, Ara h2, and Ara h3.

10. The method of claim 9, wherein said overlapping peptides each comprise an amino acid sequence of from 10 amino acids in length to 30 amino acids in length.

11. The method of claim 10 wherein each of said overlapping peptides comprises a sequence that overlaps with at least 50% of the sequence of at least one other peptide in said microarray.

12. The method of claim 11, wherein said microarray comprises an array of peptides 20 amino acids in length derived from Ara h1, Ara h2, or Ara h3, wherein each peptide has a 15 amino acid overlap with at least one other peptide derived from the same allergen.

13. The method of claim 1, wherein said determining comprises an immunoassay which comprises contacting a microarray of a plurality of sequential epitopes from said allergen with serum obtained from said subject and determining the amount of IgE bound to each epitope in said microarray using an anti-IgE antibody.

14. The method of claim 3, wherein said microarray comprises epitopes derived from a plurality of different allergens.

15. The method of claim 1, wherein said different allergens are derived from the same source.

16. The method of claim 1, wherein said different allergens are derived from different sources.

17. A method according to any of claims 1-16, wherein the allergen is a peanut allergen and the plurality of epitopes comprises an isolated peptide of residues 361-385 of SEQ ID NO:13.

18. The method of claim 1, wherein predicting the severity of an immunological response against an allergen in a subject comprises predicting an anaphylactic reaction against an allergen in the subject.

## Patentansprüche

1. Verfahren zur Vorhersage der Schwere einer immunologischen Antwort auf ein Allergen in einem Subjekt, wobei eine schwache immunologische Antwort Hautsymptome einbezieht und eine starke immunologische Antwort mehrere Organsysteme einbezieht, wobei das Verfahren ein Binden von IgE aus dem Serum des Subjekts an eine Vielzahl von Epitopen von einem Allergen und Bestimmen des Ausmaßes der Epitopbindungsdiversität des IgE aus dem Serum des Subjekts an eine Vielzahl von Epitopen an dem Allergen umfasst, wobei IgE-Bindung an vier oder mehr Epitope an dem Allergen eine schwere allergische Antwort anzeigt, wobei eine IgE-Bindung an weniger als vier Epitope eine schwache allergische Antwort anzeigt und wobei das Allergen ein Nahrungsmittelallergen ist, das für IgEvermittelte Hypersensitivität verantwortlich ist.

2. Verfahren nach Anspruch 1, wobei die Epitope von dem Allergen auf einer festen Auflage angeordnet sind.

3. Verfahren nach Anspruch 1, wobei das Binden des IgE aus dem Serum des Subjekts auf einem Mikroarray-Immuntest ausgeführt wird.

4. Verfahren nach Anspruch 1, wobei das Allergen ein Protein ist.

5. Verfahren nach Anspruch 3, wobei die Epitope des Proteins auf einer festen Auflage als eine Serie überlappender Peptidfragmente des Allergens angeordnet sind.

6. Verfahren nach Anspruch 5, wobei das IgE aus dem Serum des Subjekts an sequenzielle Epitope auf dem Allergen bindet.

7. Verfahren nach Anspruch 1, wobei das Nahrungsmittelallergen ein Allergen aus einem Nahrungsmittel ist, ausgewählt aus der Gruppe, bestehend aus einer Hülsenfrucht, einer Baumnuss, einem Cerealienkorn, einer Frucht, einem Gemüse, einem Samen, einem Fisch, einem Krustentier, einem Mollusken, Geflügel und einem Milchprodukt.

8. Verfahren nach Anspruch 7, wobei das Allergen ein Erdnussallergen ist, ausgewählt aus der Gruppe, bestehend aus Ara h1, Ara h2 und Ara h3.

9. Verfahren nach Anspruch 8, wobei das Binden des IgE aus dem Serum des Subjekts an das Erdnussallergen unter Verwendung eines Mikroarray-Immuntests ausgeführt wird, wobei der Mikroarray eine Anordnung von überlappenden Peptiden von einem oder mehreren der Allergene Ara h1, Ara h2 und Ara h3 umfasst.

10. Verfahren nach Anspruch 9, wobei die überlappenden Peptide jeweils eine Aminosäuresequenz von einer Länge von zehn Aminosäuren bis zu einer Länge von 30 Aminosäuren umfassen.

11. Verfahren nach Anspruch 10, wobei jedes der überlappenden Peptide eine Sequenz umfasst, die mit wenigstens 50% der Sequenz von wenigstens einem anderen Peptid in dem Mikroarray überlappt.

12. Verfahren nach Anspruch 11, wobei der Mikroarray eine Anordnung von Peptiden mit einer Länge von 20 Aminosäuren umfasst, abgeleitet von Ara h1, Ara h2 oder Ara h3, wobei jedes Peptid eine Überlappung von 15 Aminosäuren mit wenigstens einem anderen Peptid aufweist, das von demselben Allergen abgeleitet ist.

13. Verfahren nach Anspruch 1, wobei das Bestimmen einen Immuntest umfasst, der das Inkontaktbringen eines Mikroarray von einer Vielzahl von sequenziellen Epitopen von dem Allergen mit Serum umfasst, das von dem Subjekt erhalt ist, und Bestimmen der Menge von an jedes Epitop in dem Mikroarray gebundenem IgE, unter Verwendung eines Anti-IgE-Antikörpers.

14. Verfahren nach Anspruch 3, wobei der Mikroarray Epitope umfasst, die von einer Vielzahl von unterschiedlichen Allergenen abgeleitet sind.

15. Verfahren nach Anspruch 1, wobei die unterschiedlichen Allergene von derselben Quelle abgeleitet sind.

16. Verfahren nach Anspruch 1, wobei die unterschiedlichen Allergene von unterschiedlichen Quellen abgeleitet sind.

17. Verfahren gemäß einem der Ansprüche 1 bis 16, wobei das Allergen ein Erdnussallergen ist und die Vielzahl von Epitopen ein isoliertes Peptid der Reste 361-385 aus SEQ ID NO:13 umfasst.

18. Verfahren nach Anspruch 1, wobei das Vorhersagen der Schwere einer immunologischen Antwort gegen ein Allergen in einem Subjekt das Vorhersagen einer anaphylaktischen Reaktion gegen ein Allergen in dem Subjekt umfasst.

## Revendications

1. Procédé de prédiction de la gravité d'une réponse immunologique contre un allergène chez un sujet, dans lequel une réponse immunologique modérée comprend des symptômes cutanés et une réponse immunologique grave implique de multiples systèmes d'organes, le procédé comprenant les étapes consistant à lier l'IgE provenant du sérum dudit sujet à une pluralité d'épitopes provenant d'un allergène et à déterminer le degré de diversité des liaisons aux épitopes par l'IgE provenant du sérum dudit sujet se liant à une pluralité d'épitopes sur ledit allergène, dans lequel la liaison de l'IgE à quatre ou plus de quatre épitopes sur ledit allergène indique une réponse allergique grave, dans lequel la liaison de l'IgE à moins de quatre épitopes indique une réponse allergique modérée, et dans lequel l'allergène est un allergène alimentaire responsable d'une hypersensibilité induite par l'IgE.

2. Procédé selon la revendication 1, dans lequel les épitopes provenant dudit allergène sont disposés en un jeu ordonné d'échantillons sur un support solide.

3. Procédé selon la revendication 1, dans lequel ladite liaison de ladite IgE provenant du sérum dudit sujet est réalisée par le biais d'un dosage immunologique sur une micro-puce.

4. Procédé selon la revendication 1, dans lequel ledit allergène est une protéine.

5. Procédé selon la revendication 3, dans lequel les épitopes de la protéine sont disposés en un jeu ordonné d'échantillons sur un support solide sous la forme d'une série de fragments peptidiques se chevauchant dudit allergène.

6. Procédé selon la revendication 5, dans lequel ladite IgE provenant du sérum dudit sujet se lie à des épitopes séquentiels sur ledit allergène.

7. Procédé selon la revendication 1, dans lequel ledit allergène alimentaire est un allergène provenant d'un produit alimentaire choisi dans le groupe comprenant un légume, une noix, un grain de céréale, un fruit, un végétal, une graine, un poisson, un crustacé, un mollusque, une volaille et un produit laitier.

8. Procédé selon la revendication 7, dans lequel ledit allergène est un allergène d'arachides choisis dans le groupe comprenant Ara h1, Ara h2 et Ara h3.

9. Procédé selon la revendication 8, dans lequel ladite liaison de ladite IgE provenant du sérum dudit sujet au dit allergène d'arachides est réalisée en utilisant un dosage immunologique sur une micro-puce, dans lequel ladite micro-puce comprend un jeu ordonné de peptides se chevauchant provenant d'un ou de plusieurs allergènes parmi Ara h1, Ara h2, et Ara h3.

10. Procédé selon la revendication 9, dans lequel lesdits peptides se chevauchant comprennent chacun une séquence d'acides aminés allant de 10 acides aminés de longueur à 30 acides aminés de longueur.

11. Procédé selon la revendication 10, dans lequel chacun desdits peptides se chevauchant comprend une séquence se chevauchant avec au moins 50 % de la séquence d'au moins un autre peptide sur ladite micro-puce.

12. Procédé selon la revendication 11, dans lequel ladite micro-puce comprend un jeu ordonné de peptides de 20 acides aminés de longueur dérivés de Ara h1, Ara h2, ou Ara h3, dans lequel chaque peptide se chevauche sur 15 acides aminés avec au moins un autre peptide dérivé du même allergène.

13. Procédé selon la revendication 1, dans lequel ladite étape de détermination comprend un dosage immunologique comprenant la mise en contact d'une micro-puce d'une pluralité d'épitopes séquentiels provenant dudit allergène avec du sérum obtenu à partir dudit sujet et la détermination de la quantité d'IgE liée à chaque épitope dans ladite micro-puce en utilisant un anticorps anti-IgE.

14. Procédé selon la revendication 3, dans lequel ladite micro-puce comprend des épitopes dérivés d'une pluralité d'allergènes différents.

15. Procédé selon la revendication 1, dans lequel lesdits différents allergènes sont dérivés de la même source.

16. Procédé selon la revendication 1, dans lequel lesdits différents allergènes sont dérivés de sources différentes.

17. Procédé selon l'une quelconque des revendications 1 à 16, dans lequel l'allergène est un allergène d'arachides et la pluralité d'épitopes comprend un peptide isolé des résidus 361 à 385 de la SEQ ID No. : 13.

18. Procédé selon la revendication 1, dans lequel l'étape de prédiction de la gravité d'une réponse immunologique contre un allergène chez un sujet comprend la prédiction d'une réaction anaphylactique contre un allergène chez le sujet.


### SEQUENCE LISTING

<110> Sampson et al.
<120> METHODS OF DETERMINING ALLERGEN RESPONSE USING MICROARRAY IMMUNOASSAY TECHNIQUES
<130> 29636/40002B
<150> US 60/559,825
   <151> 2004-04-06
<150> US 60/620,923
   <151> 2004-10-21
<160> 13
<170> PatentIn version 3.2
<210> 1
   <211> 626
   <212> PRT
   <213> Arachis hypogaea
<400> 1
   Met Arg Gly Arg Val Ser Pro Leu Met Leu Leu Leu Gly Ile Leu Val
   1               5                   10                  15
   Leu Ala Ser Val Ser Ala Thr His Ala Lys Ser Ser Pro Tyr Gln Lys
               20                  25                  30
   Lys Thr Glu Asn Pro Cys Ala Gln Arg Cys Leu Gln Ser Cys Gln Gln
           35                  40                  45
   Glu Pro Asp Asp Leu Lys Gln Lys Ala Cys Glu Ser Arg Cys Thr Lys
       50                  55                  60
   Leu Glu Tyr Asp Pro Arg Cys Val Tyr Asp Pro Arg Gly His Thr Gly
   65                  70                  75                  80
   Thr Thr Asn Gln Arg Ser Pro Pro Gly Glu Arg Thr Arg Gly Arg Gln
                   85                  90                  95
   Pro Gly Asp Tyr Asp Asp Asp Arg Arg Gln Pro Arg Arg Glu Glu Gly
               100                 105                 110
   Gly Arg Trp Gly Pro Ala Gly Pro Arg Glu Arg Glu Arg Glu Glu Asp
           115                 120                 125
   Trp Arg Gln Pro Arg Glu Asp Trp Arg Arg Pro Ser His Gln Gln Pro
       130                 135                 140
   Arg Lys Ile Arg Pro Glu Gly Arg Glu Gly Glu Gln Glu Trp Gly Thr
   145 150 155 160
   Pro Gly Ser His Val Arg Glu Glu Thr Ser Arg Asn Asn Pro Phe Tyr
   165 170 175
   Phe Pro Ser Arg Arg Phe Ser Thr Arg Tyr Gly Asn Gln Asn Gly Arg
   180 185 190
   Ile Arg Val Leu Gln Arg Phe Asp Gln Arg Ser Arg Gln Phe Gln Asn
   195 200 205
   Leu Gln Asn His Arg Ile Val Gln Ile Glu Ala Lys Pro Asn Thr Leu
   210 215 220
   Val Leu Pro Lys His Ala Asp Ala Asp Asn Ile Leu Val Ile Gln Gln
   225 230 235 240
   Gly Gln Ala Thr Val Thr Val Ala Asn Gly Asn Asn Arg Lys Ser Phe
   245 250 255
   Asn Leu Asp Glu Gly His Ala Leu Arg Ile Pro Ser Gly Phe Ile Ser
   260 265 270
   Tyr Ile Leu Asn Arg His Asp Asn Gln Asn Leu Arg Val Ala Lys Ile
   275 280 285
   Ser Met Pro Val Asn Thr Pro Gly Gln Phe Glu Asp Phe Phe Pro Ala
   290 295 300
   Ser Ser Arg Asp Gln Ser Ser Tyr Leu Gln Gly Phe Ser Arg Asn Thr
   305 310 315 320
   Leu Glu Ala Ala Phe Asn Ala Glu Phe Asn Glu Ile Arg Arg Val Leu
   325 330 335
   Leu Glu Glu Asn Ala Gly Gly Glu Gln Glu Glu Arg Gly Gln Arg Arg
   340 345 350
   Trp Ser Thr Arg Ser Ser Glu Asn Asn Glu Gly Val Ile Val Lys Val
   355 360 365
   Ser Lys Glu His Val Glu Glu Leu Thr Lys His Ala Lys Ser Val Ser
   370 375 380
   Lys Lys Gly Ser Glu Glu Glu Gly Asp Ile Thr Asn Pro Ile Asn Leu
   385 390 395 400
   Arg Glu Gly Glu Pro Asp Leu Ser Asn Asn Phe Gly Lys Leu Phe Glu
                   405                 410                 415
   Val Lys Pro Asp Lys Lys Asn Pro Gln Leu Gln Asp Leu Asp Met Met
               420                 425                 430
   Leu Thr Cys Val Glu Ile Lys Glu Gly Ala Leu Met Leu Pro His Phe
           435                 440                 445
   Asn Ser Lys Ala Met Val Ile Val Val Val Asn Lys Gly Thr Gly Asn
       450                 455                 460
   Leu Glu Leu Val Ala Val Arg Lys Glu Gln Gln Gln Arg Gly Arg Arg
   465                 470                 475                 480
   Glu Glu Glu Glu Asp Glu Asp Glu Glu Glu Glu Gly Ser Asn Arg Glu
                   485                 490                 495
   Val Arg Arg Tyr Thr Ala Arg Leu Lys Glu Gly Asp Val Phe Ile Met
               500                 505                 510
   Pro Ala Ala His Pro Val Ala Ile Asn Ala Ser Ser Glu Leu His Leu
           515                 520                 525
   Leu Gly Phe Gly Ile Asn Ala Glu Asn Asn His Arg Ile Phe Leu Ala
       530                 535                 540
   Gly Asp Lys Asp Asn Val Ile Asp Gln Ile Glu Lys Gln Ala Lys Asp
   545                 550                 555                 560
   Leu Ala Phe Pro Gly Ser Gly Glu Gln Val Glu Lys Leu Ile Lys Asn
                   565                 570                 575
   Gln Lys Glu Ser His Phe Val Ser Ala Arg Pro Gln Ser Gln Ser Gln
               580                 585                 590
   Ser Pro Ser Ser Pro Glu Lys Glu Ser Pro Glu Lys Glu Asp Gln Glu
           595                 600                 605
   Glu Glu Asn Gln Gly Gly Lys Gly Pro Leu Leu Ser Ile Leu Lys Ala
       610                 615                 620
   Phe Asn
   625
<210> 2
   <211> 169
   <212> PRT
   <213> Arachis hypogaea
<400> 2
   Leu Thr Ile Leu Val Ala Pro Ala Leu Phe Leu Leu Ala Ala His Ala
   1               5                   10                  15
   Ser Ala Arg Gln Gln Trp Glu Leu Gln Gly Asp Arg Arg Cys Gln Ser
               20                  25                  30
   Gln Leu Glu Arg Ala Asn Leu Arg Pro Cys Glu Gln His Leu Met Gln
           35                  40                  45
   Lys Ile Gln Arg Asp Glu Asp Ser Tyr Gly Arg Asp Pro Tyr Ser Pro
       50                  55                  60
   Ser Gln Asp Pro Tyr Ser Pro Ser Gln Asp Pro Asp Arg Arg Asp Pro
   65                  70                  75                  80
   Tyr Ser Pro Ser Pro Tyr Asp Arg Arg Gly Ala Gly Ser Ser Gln His
                   85                  90                  95
   Gln Glu Arg Cys Cys Asn Glu Leu Asn Glu Phe Glu Asn Asn Gln Arg
               100                 105                 110
   Cys Met Cys Glu Ala Leu Gln Gln Ile Met Glu Asn Gln Ser Asp Arg
           115                 120                 125
   Leu Gln Gly Arg Gln Gln Glu Gln Gln Phe Lys Arg Glu Leu Arg Asn
       130                 135                 140
   Leu Pro Gln Gln Cys Gly Leu Arg Ala Pro Gln Arg Cys Asp Leu Glu
   145                 150                 155                 160
   Val Glu Ser Gly Gly Arg Asp Arg Tyr
                   165
<210> 3
   <211> 156
   <212> PRT
   <213> Arachis hypogaea
<400> 3
   Met Ala Lys Leu Thr Ile Leu Val Ala Leu Ala Leu Phe Leu Leu Ala
   1               5                   10                  15
   Ala His Ala Ser Ala Arg Gln Gln Trp Glu Leu Gln Gly Asp Arg Arg
               20                  25                  30
   Cys Gln Ser Gln Leu Glu Arg Ala Asn Leu Arg Pro Cys Glu Gln His
           35                  40                  45
   Leu Met Gln Lys Ile Gln Arg Asp Glu Asp Ser Tyr Glu Arg Asp Pro
       50                  55                  60
   Tyr Ser Pro Ser Gln Asp Pro Tyr Ser Pro Ser Pro Tyr Asp Arg Arg
   65                  70                  75                  80
   Gly Ala Gly Ser Ser Gln His Gln Glu Arg Cys Cys Asn Glu Leu Asn
                   85                  90                  95
   Glu Phe Glu Asn Asn Gln Arg Cys Met Cys Glu Ala Leu Gln Gln Ile
               100                 105                 110
   Met Glu Asn Gln Ser Asp Arg Leu Gln Gly Arg Gln Gln Glu Gln Gln
           115                 120                 125
   Phe Lys Arg Glu Leu Arg Asn Leu Pro Gln Gln Cys Gly Leu Arg Ala
       130                 135                 140
   Pro Gln Arg Cys Asp Leu Asp Val Glu Ser Gly Gly
   145                 150                 155
<210> 4
   <211> 538
   <212> PRT
   <213> Arachis hypogaea
<400> 4
   Met Gly Lys Leu Leu Ala Leu Ser Val Cys Phe Cys Phe Leu Val Leu
   1 5 10 15
   Gly Ala Ser Ser Ile Ser Phe Arg Gln Gln Pro Glu Glu Asn Ala Cys
   20 25 30
   Gln Phe Gln Arg Leu Asn Ala Gln Arg Pro Asp Asn Arg Ile Glu Ser
   35 40 45
   Glu Gly Gly Tyr Ile Glu Thr Trp Asn Pro Asn Asn Gln Glu Phe Glu
   50 55 60
   Cys Ala Gly Val Ala Leu Ser Arg Leu Val Leu Arg Arg Asn Ala Leu
   65 70 75 80
   Arg Arg Pro Phe Tyr Ser Asn Ala Pro Gln Glu Ile Phe Ile Gln Gln
   85 90 95
   Gly Arg Gly Tyr Phe Gly Leu Ile Phe Pro Gly Cys Pro Ser Thr Tyr
               100                 105                 110
   Glu Glu Pro Ala Gln Gln Gly Arg Arg His Gln Ser Gln Arg Pro Pro
           115                 120                 125
   Arg Arg Phe Gln Gly Gln Asp Gln Ser Gln Gln Gln Gln Asp Ser His
       130                 135                 140
   Gln Lys Val His Arg Phe Asp Glu Gly Asp Leu Ile Ala Val Pro Thr
   145                 150                 155                 160
   Gly Val Ala Phe Trp Met Tyr Asn Asp His Asp Thr Asp Val Val Ala
                   165                 170                 175
   Val Ser Leu Thr Asp Thr Asn Asn Asn Asp Asn Gln Leu Asp Gln Phe
               180                 185                 190
   Pro Arg Arg Phe Asn Leu Ala Gly Asn His Glu Gln Glu Phe Leu Arg
           195                 200                 205
   Tyr Gln Gln Gln Ser Arg Arg Arg Ser Leu Pro Tyr Ser Pro Tyr Ser
       210                 215                 220
   Pro Gln Thr Gln Pro Lys Gln Glu Asp Arg Glu Phe Ser Pro Arg Gly
   225                 230                 235                 240
   Gln His Gly Arg Arg Glu Arg Ala Gly Gln Glu Gln Glu Asn Glu Gly
                   245                 250                 255
   Gly Asn Ile Phe Ser Gly Phe Thr Pro Glu Phe Leu Ala Gln Ala Phe
               260                 265                 270
   Gln Val Asp Asp Arg Gln Ile Leu Gln Asn Leu Arg Gly Glu Asn Glu
           275                 280                 285
   Ser Asp Glu Gln Gly Ala Ile Val Thr Val Arg Gly Gly Leu Arg Ile
       290                 295                 300
   Leu Ser Pro Asp Arg Lys Arg Arg Gln Gln Tyr Glu Arg Pro Asp Glu
   305                 310                 315                 320
   Glu Glu Glu Tyr Asp Glu Asp Glu Tyr Glu Tyr Asp Glu Glu Glu Arg
                   325                 330                 335
   Gln Gln Asp Arg Arg Arg Gly Arg Gly Ser Arg Gly Ser Gly Asn Gly
               340                 345                 350
   Ile Glu Glu Thr Ile Cys Thr Ala Ser Phe Lys Lys Asn Ile Gly Arg
   355 360 365
   Asn Arg Ser Pro Asp Ile Tyr Asn Pro Gln Ala Gly Ser Leu Lys Thr
   370 375 380
   Ala Asn Glu Leu Gln Leu Asn Leu Leu Ile Leu Arg Trp Leu Gly Leu
   385 390 395 400
   Ser Ala Glu Tyr Gly Asn Leu Tyr Arg Asn Ala Leu Phe Val Pro His
   405 410 415
   Tyr Asn Thr Asn Ala His Ser Ile Ile Tyr Ala Leu Arg Gly Arg Ala
   420 425 430
   His Val Gln Val Val Asp Ser Asn Gly Asp Arg Val Phe Asp Glu Glu
   435 440 445
   Leu Gln Glu Gly His Val Leu Val Val Pro Gln Asn Phe Ala Val Ala
   450 455 460
   Gly Lys Ser Gln Ser Glu Asn Phe Glu Tyr Val Ala Phe Lys Thr Asp
   465 470 475 480
   Ser Arg Pro Ser Ile Ala Asn Leu Ala Gly Glu Asn Ser Phe Ile Asp
   485 490 495
   Asn Leu Pro Glu Glu Val Val Ala Asn Ser Tyr Gly Leu Pro Arg Glu
   500 505 510
   Gln Ala Arg Gln Leu Lys Asn Asn Asn Pro Phe Lys Phe Phe Val Pro
   515 520 525
   Pro Ser Glu Gln Ser Leu Arg Ala Val Ala
   530 535
<210> 5
   <211> 530
   <212> PRT
   <213> Arachis hypogaea
<400> 5
   Met Ala Lys Leu Leu Glu Leu Ser Phe Cys Phe Cys Phe Leu Val Leu
   1               5                   10                  15
   Gly Ala Ser Ser Ile Ser Phe Arg Gln Gln Pro Glu Glu Asn Ala Cys
               20                  25                  30
   Gln Phe Gln Arg Leu Asn Ala Gln Arg Pro Asp Asn Arg Ile Glu Ser
   35 40 45
   Glu Gly Gly Tyr Ile Glu Thr Trp Asn Pro Asn Asn Gln Glu Phe Glu
   50 55 60
   Cys Ala Gly Val Ala Leu Ser Arg Leu Val Leu Arg Arg Asn Ala Leu
   65 70 75 80
   Arg Arg Pro Phe Tyr Ser Asn Ala Pro Gln Glu Ile Phe Ile Gln Gln
   85 90 95
   Gly Arg Gly Tyr Phe Gly Leu Ile Phe Pro Gly Cys Pro Ser Thr Tyr
   100 105 110
   Glu Glu Pro Ala Gln Gln Gly Arg Arg Tyr Gln Ser Gln Arg Pro Pro
   115 120 125
   Arg Arg Leu Gln Glu Glu Asp Gln Ser Gln Gln Gln Gln Asp Ser His
   130 135 140
   Gln Lys Val His Arg Phe Asn Glu Gly Asp Leu Ile Ala Val Pro Thr
   145 150 155 160
   Gly Val Ala Phe Trp Leu Tyr Asn Asp His Asp Thr Asp Val Val Ala
   165 170 175
   Val Ser Leu Thr Asp Thr Asn Asn Asn Asp Asn Gln Leu Asp Gln Phe
   180 185 190
   Pro Arg Arg Phe Asn Leu Ala Gly Asn His Glu Gln Glu Phe Leu Arg
   195 200 205
   Tyr Gln Gln Gln Ser Arg Gln Ser Arg Arg Arg Ser Leu Pro Tyr Ser
   210 215 220
   Pro Tyr Ser Pro His Ser Arg Pro Arg Arg Glu Glu Arg Glu Phe Arg
   225 230 235 240
   Pro Arg Gly Gln His Ser Arg Arg Glu Arg Ala Gly Gln Glu Glu Glu
   245 250 255
   Asp Glu Gly Gly Asn Ile Phe Ser Gly Phe Thr Pro Glu Phe Leu Glu
   260 265 270
   Gln Ala Phe Gln Val Asp Asp Arg Gln Ile Val Gln Asn Leu Trp Gly
   275 280 285
   Glu Asn Glu Ser Glu Glu Glu Gly Ala Ile Val Thr Val Arg Gly Gly
   290 295 300
   Leu Arg Ile Leu Ser Pro Asp Gly Thr Arg Gly Ala Asp Glu Glu Glu
   305 310 315 320
   Glu Tyr Asp Glu Asp Gln Tyr Glu Tyr His Glu Gln Asp Gly Arg Arg
   325 330 335
   Gly Arg Gly Ser Arg Gly Gly Gly Asn Gly Ile Glu Glu Thr Ile Cys
   340 345 350
   Thr Ala Cys Val Lys Lys Asn Ile Gly Gly Asn Arg Ser Pro His Ile
   355 360 365
   Tyr Asp Pro Gln Arg Trp Phe Thr Gln Asn Cys His Asp Leu Asn Leu
   370 375 380
   Leu Ile Leu Arg Trp Leu Gly Leu Ser Ala Glu Tyr Gly Asn Leu Tyr
   385 390 395 400
   Arg Asn Ala Leu Phe Val Pro His Tyr Asn Thr Asn Ala His Ser Ile
   405 410 415
   Ile Tyr Ala Leu Arg Gly Arg Ala His Val Gln Val Val Asp Ser Asn
   420 425 430
   Gly Asn Arg Val Tyr Asp Glu Glu Leu Gln Glu Gly His Val Leu Val
   435 440 445
   Val Pro Gln Asn Phe Ala Val Ala Gly Lys Ser Gln Ser Glu Asn Phe
   450 455 460
   Glu Tyr Val Ala Phe Lys Thr Asp Ser Arg Pro Ser Ile Ala Asn Phe
   465 470 475 480
   Ala Gly Glu Asn Ser Phe Ile Asp Asn Leu Pro Glu Glu Val Val Ala
   485 490 495
   Asn Ser Tyr Gly Leu Pro Arg Glu Gln Ala Arg Gln Leu Lys Asn Asn
   500 505 510
   Asn Pro Phe Lys Phe Phe Val Pro Pro Phe Gln Gln Ser Pro Arg Ala
   515 520 525
   Val Ala
   530
<210> 6
   <211> 530
   <212> PRT
   <213> Arachis hypogaea
<400> 6
   Met Ala Lys Leu Leu Glu Leu Ser Phe Cys Phe Cys Phe Leu Val Leu
   1               5                   10                  15
   Gly Ala Ser Ser Ile Ser Phe Arg Gln Gln Pro Glu Glu Asn Ala Cys
               20                  25                  30
   Gln Phe Gln Arg Leu Asn Ala Gln Arg Pro Asp Asn Arg Ile Glu Ser
           35                  40                  45
   Glu Gly Gly Tyr Ile Glu Thr Trp Asn Pro Asn Asn Gln Glu Phe Glu
       50                  55                  60
   Cys Ala Gly Val Ala Leu Ser Arg Leu Val Leu Arg Arg Asn Ala Leu
   65                  70                  75                  80
   Arg Arg Pro Phe Tyr Ser Asn Ala Pro Gln Glu Ile Phe Ile Gln Gln
                   85                  90                  95
   Gly Arg Gly Tyr Phe Gly Leu Ile Phe Pro Gly Cys Pro Ser Thr Tyr
               100                 105                 110
   Glu Glu Pro Ala Gln Gln Gly Arg Arg Tyr Gln Ser Gln Arg Pro Pro
           115                 120                 125
   Arg Arg Leu Gln Glu Glu Asp Gln Ser Gln Gln Gln Gln Asp Ser His
       130                 135                 140
   Gln Lys Val His Arg Phe Asn Glu Gly Asp Leu Ile Ala Val Pro Thr
   145                 150                 155                 160
   Gly Val Ala Phe Trp Leu Tyr Asn Asp His Asp Thr Asp Val Val Ala
                   165                 170                 175
   Val Ser Leu Thr Asp Thr Asn Asn Asn Asp Asn Gln Leu Asp Gln Phe
               180                 185                 190
   Pro Arg Arg Phe Asn Leu Ala Gly Asn His Glu Gln Glu Phe Leu Arg
           195                 200                 205
   Tyr Gln Gln Gln Ser Arg Gln Ser Arg Arg Arg Ser Leu Pro Tyr Ser
       210                 215                 220
   Pro Tyr Ser Pro His Ser Arg Pro Arg Arg Glu Glu Arg Glu Phe Arg
   225                 230                 235                 240
   Pro Arg Gly Gln His Ser Arg Arg Glu Arg Ala Gly Gln Glu Glu Glu
                   245                 250                 255
   Asp Glu Gly Gly Asn Ile Phe Ser Gly Phe Thr Pro Glu Phe Leu Glu
               260                 265                 270
   Gln Ala Phe Gln Val Asp Asp Arg Gln Ile Val Gln Asn Leu Trp Gly
           275                 280                 285
   Glu Asn Glu Ser Glu Glu Glu Gly Ala Ile Val Thr Val Arg Gly Gly
       290                 295                 300
   Leu Arg Ile Leu Ser Pro Asp Gly Thr Arg Gly Ala Asp Glu Glu Glu
   305                 310                 315                 320
   Glu Tyr Asp Glu Asp Gln Tyr Glu Tyr His Glu Gln Asp Gly Arg Arg
                   325                 330                 335
   Gly Arg Gly Ser Arg Gly Gly Gly Asn Gly Ile Glu Glu Thr Ile Cys
               340                 345                 350
   Thr Ala Cys Val Lys Lys Asn Ile Gly Gly Asn Arg Ser Pro His Ile
           355                 360                 365
   Tyr Asp Pro Gln Arg Trp Phe Thr Gln Asn Cys His Asp Leu Asn Leu
       370                 375                 380
   Leu Ile Leu Arg Trp Leu Gly Leu Ser Ala Glu Tyr Gly Asn Leu Tyr
   385                 390                 395                 400
   Arg Asn Ala Leu Phe Val Pro His Tyr Asn Thr Asn Ala His Ser Ile
                   405                 410                 415
   Ile Tyr Ala Leu Arg Gly Arg Ala His Val Gln Val Val Asp Ser Asn
               420                 425                 430
   Gly Asn Arg Val Tyr Asp Glu Glu Leu Gln Glu Gly His Val Leu Val
           435                 440                 445
   Val Pro Gln Asn Phe Ala Val Ala Gly Lys Ser Gln Ser Glu Asn Phe
       450                 455                 460
   Glu Tyr Val Ala Phe Lys Thr Asp Ser Arg Pro Ser Ile Ala Asn Phe
   465                 470                 475                 480
   Ala Gly Glu Asn Ser Phe Ile Asp Asn Leu Pro Glu Glu Val Val Ala
                   485                 490                 495
   Asn Ser Tyr Gly Leu Pro Arg Glu Gln Ala Arg Gln Leu Lys Asn Asn
               500                 505                 510
   Asn Pro Phe Lys Phe Phe Val Pro Pro Phe Gln Gln Ser Pro Arg Ala
           515                 520                 525
   Val Ala
       530
<210> 7
   <211> 131
   <212> PRT
   <213> Arachis hypogaea
<400> 7
   Met Ser Trp Gln Thr Tyr Val Asp Asn His Leu Leu Cys Glu Ile Glu
   1               5                   10                  15
   Gly Asp His Leu Ser Ser Ala Ala Ile Leu Gly Gln Asp Gly Gly Val
               20                  25                  30
   Trp Ala Gln Ser Ser His Phe Pro Gln Phe Lys Pro Glu Glu Ile Thr
           35                  40                  45
   Ala Ile Met Asn Asp Phe Ala Glu Pro Gly Ser Leu Ala Pro Thr Gly
       50                  55                  60
   Leu Tyr Leu Gly Gly Thr Lys Tyr Met Val Ile Gln Gly Glu Pro Gly
   65                  70                  75                  80
   Ala Ile Ile Pro Gly Lys Lys Gly Pro Gly Gly Val Thr Ile Glu Lys
                   85                  90                  95
   Thr Asn Gln Ala Leu Ile Ile Gly Ile Tyr Asp Lys Pro Met Thr Pro
               100                 105                 110
   Gly Gln Cys Asn Met Ile Val Glu Arg Leu Gly Asp Tyr Leu Ile Asp
           115                 120                 125
   Thr Gly Leu
       130
<210> 8
   <211> 131
   <212> PRT
   <213> Arachis hypogaea
<400> 8
   Met Ser Trp Gln Thr Tyr Val Asp Asn His Leu Leu Cys Glu Ile Glu
   1               5                   10                  15
   Gly Asp His Leu Ser Ser Ala Ala Ile Leu Gly Gln Asp Gly Gly Val
               20                  25                  30
   Trp Ala Gln Ser Ser His Phe Pro Gln Phe Lys Pro Glu Glu Ile Thr
           35                  40                  45
   Ala Ile Met Asn Asp Phe Ala Glu Pro Gly Ser Leu Ala Pro Thr Gly
       50                  55                  60
   Leu Tyr Leu Gly Gly Thr Lys Tyr Met Val Ile Gln Gly Glu Pro Gly
   65                  70                  75                  80
   Ala Ile Ile Pro Gly Lys Lys Gly Pro Gly Gly Val Thr Ile Glu Lys
                   85                  90                  95
   Thr Asn Gln Ala Leu Ile Ile Gly Ile Tyr Asp Lys Pro Met Thr Pro
               100                 105                 110
   Gly Gln Cys Asn Met Ile Val Glu Arg Leu Gly Asp Tyr Leu Ile Asp
           115                 120                 125
   Thr Gly Leu
       130
<210> 9
   <211> 129
   <212> PRT
   <213> Arachia hypogaea
<400> 9
   Ala His Ala Ser Ala Met Arg Arg Glu Arg Gly Arg Gln Gly Asp Ser
   1               5                   10                  15
   Ser Ser Cys Glu Arg Gln Val Asp Gly Val Asn Leu Lys Pro Cys Glu
               20                  25                  30
   Gln His Ile Met Gln Arg Ile Met Gly Glu Gln Glu Gln Tyr Asp Ser
           35                  40                  45
   Tyr Asn Phe Gly Ser Thr Arg Ser Ser Asp Gln Gln Gln Arg Cys Cys
       50                  55                  60
   Asp Glu Leu Asn Glu Met Glu Asn Thr Gln Arg Cys Met Cys Glu Ala
   65                  70                  75                  80
   Leu Gln Gln Ile Met Glu Asn Gln Cys Asp Gly Leu Gln Asp Arg Gln
                   85                  90                  95
   Met Val Gln His Phe Lys Arg Glu Leu Met Asn Leu Pro Gln Gln Cys
               100                 105                 110
   Asn Phe Gly Ala Pro Gln Arg Cys Asp Leu Asp Val Ser Gly Gly Arg
           115                 120                 125
   Cys
<210> 10
   <211> 160
   <212> PRT
   <213> Arachis hypogaea
<400> 10
   Met Met Val Lys Leu Ser Ile Leu Val Ala Leu Leu Gly Ala Leu Leu
   1               5                   10                  15
   Val Val Ala Ser Ala Thr Arg Trp Asp Pro Asp Arg Gly Ser Arg Gly
               20                  25                  30
   Ser Arg Trp Asp Ala Pro Ser Arg Gly Asp Asp Gln Cys Gln Arg Gln
           35                  40                  45
   Leu Gln Arg Ala Asn Leu Arg Pro Cys Glu Glu His Met Arg Arg Arg
       50                  55                  60
   Val Glu Gln Glu Gln Glu Gln Glu Gln Asp Glu Tyr Pro Tyr Ser Arg
   65                  70                  75                  80
   Arg Gly Ser Arg Gly Arg Gln Pro Gly Glu Ser Asp Glu Asn Gln Glu
                   85                  90                  95
   Gln Arg Cys Cys Asn Glu Leu Asn Arg Phe Gln Asn Asn Gln Arg Cys
               100                 105                 110
   Met Cys Gln Ala Leu Gln Gln Ile Leu Gln Asn Gln Ser Phe Trp Val
           115                 120                 125
   Pro Ala Gly Gln Glu Pro Val Ala Ser Asp Gly Glu Gly Ala Gln Glu
       130                 135                 140
   Leu Ala Pro Glu Leu Arg Val Gln Val Thr Lys Pro Leu Arg Pro Leu
   145                 150                 155                 160
<210> 11
   <211> 157
   <212> PRT
   <213> Arachis hypogaea
<400> 11
   Met Gly Val Phe Thr Phe Glu Asp Glu Ile Thr Ser Thr Val Pro Pro
   1 5 10 15
   Ala Lys Leu Tyr Asn Ala Met Lys Asp Ala Asp Ser Ile Thr Pro Lys
   20 25 30
   Ile Ile Asp Asp Val Lys Ser Val Glu Ile Val Glu Gly Asn Gly Gly
   35 40 45
   Pro Gly Thr Ile Lys Lys Leu Thr Ile Val Glu Asp Gly Glu Thr Lys
   50 55 60
   Phe Ile Leu His Lys Val Glu Ser Ile Asp Glu Ala Asn Tyr Ala Tyr
   65 70 75 80
   Asn Tyr Ser Val Val Gly Gly Val Ala Leu Pro Pro Thr Ala Glu Lys
   85 90 95
   Ile Thr Phe Glu Thr Lys Leu Val Glu Gly Pro Asn Gly Gly Ser Ile
   100 105 110
   Gly Lys Leu Thr Leu Lys Tyr His Thr Lys Gly Asp Ala Lys Pro Asp
   115 120 125
   Glu Glu Glu Leu Lys Lys Gly Lys Ala Lys Gly Glu Gly Leu Phe Arg
   130 135 140
   Ala Ile Glu Gly Tyr Val Leu Ala Asn Pro Thr Gln Tyr
   145 150 155
<210> 12
   <211> 626
   <212> PRT
   <213> Arachis hypogaea
<400> 12
   Met Arg Gly Arg Val Ser Pro Leu Met Leu Leu Leu Gly Ile Leu Val
   1 5 10 15
   Leu Ala Ser Val Ser Ala Thr His Ala Lys Ser Ser Pro Tyr Gln Lys
   20 25 30
   Lys Thr Glu Asn Pro Cys Ala Gln Arg Cys Leu Gln Ser Cys Gln Gln
   35 40 45
   Glu Pro Asp Asp Leu Lys Gln Lys Ala Cys Glu Ser Arg Cys Thr Lys
   50 55 60
   Leu Glu Tyr Asp Pro Arg Cys Val Tyr Asp Pro Arg Gly His Thr Gly
   65 70 75 80
   Thr Thr Asn Gln Arg Ser Pro Pro Gly Glu Arg Thr Arg Gly Arg Gln
   85 90 95
   Pro Gly Asp Tyr Asp Asp Asp Arg Arg Gln Pro Arg Arg Glu Glu Gly
   100 105 110
   Gly Arg Trp Gly Pro Ala Gly Pro Arg Glu Arg Glu Arg Glu Glu Asp
   115 120 125
   Trp Arg Gln Pro Arg Glu Asp Trp Arg Arg Pro Ser His Gln Gln Pro
   130 135 140
   Arg Lys Ile Arg Pro Glu Gly Arg Glu Gly Glu Gln Glu Trp Gly Thr
   145 150 155 160
   Pro Gly Ser His Val Arg Glu Glu Thr Ser Arg Asn Asn Pro Phe Tyr
   165 170 175
   Phe Pro Ser Arg Arg Phe Ser Thr Arg Tyr Gly Asn Gln Asn Gly Arg
   180 185 190
   Ile Arg Val Leu Gln Arg Phe Asp Gln Arg Ser Arg Gln Phe Gln Asn
   195 200 205
   Leu Gln Asn His Arg Ile Val Gln Ile Glu Ala Lys Pro Asn Thr Leu
   210 215 220
   Val Leu Pro Lys His Ala Asp Ala Asp Asn Ile Leu Val Ile Gln Gln
   225 230 235 240
   Gly Gln Ala Thr Val Thr Val Ala Asn Gly Asn Asn Arg Lys Ser Phe
   245 250 255
   Asn Leu Asp Glu Gly His Ala Leu Arg Ile Pro Ser Gly Phe Ile Ser
   260 265 270
   Tyr Ile Leu Asn Arg His Asp Asn Gln Asn Leu Arg Val Ala Lys Ile
   275 280 285
   Ser Met Pro Val Asn Thr Pro Gly Gln Phe Glu Asp Phe Phe Pro Ala
   290 295 300
   Ser Ser Arg Asp Gln Ser Ser Tyr Leu Gln Gly Phe Ser Arg Asn Thr
   305 310 315 320
   Leu Glu Ala Ala Phe Asn Ala Glu Phe Asn Glu Ile Arg Arg Val Leu
   325 330 335
   Leu Glu Glu Asn Ala Gly Gly Glu Gln Glu Glu Arg Gly Gln Arg Arg
   340 345 350
   Trp Ser Thr Arg Ser Ser Glu Asn Asn Glu Gly Val Ile Val Lys Val
   355 360 365
   Ser Lys Glu His Val Glu Glu Leu Thr Lys His Ala Lys Ser Val Ser
   370 375 380
   Lys Lys Gly Ser Glu Glu Glu Gly Asp Ile Thr Asn Pro Ile Asn Leu
   385 390 395 400
   Arg Glu Gly Glu Pro Asp Leu Ser Asn Asn Phe Gly Lys Leu Phe Glu
   405 410 415
   Val Lys Pro Asp Lys Lys Asn Pro Gln Leu Gln Asp Leu Asp Met Met
   420 425 430
   Leu Thr Cys Val Glu Ile Lys Glu Gly Ala Leu Met Leu Pro His Phe
   435 440 445
   Asn Ser Lys Ala Met Val Ile Val Val Val Asn Lys Gly Thr Gly Asn
   450 455 460
   Leu Glu Leu Val Ala Val Arg Lys Glu Gln Gln Gln Arg Gly Arg Arg
   465 470 475 480
   Glu Glu Glu Glu Asp Glu Asp Glu Glu Glu Glu Gly Ser Asn Arg Glu
   485 490 495
   Val Arg Arg Tyr Thr Ala Arg Leu Lys Glu Gly Asp Val Phe Ile Met
   500 505 510
   Pro Ala Ala His Pro Val Ala Ile Asn Ala Ser Ser Glu Leu His Leu
   515 520 525
   Leu Gly Phe Gly Ile Asn Ala Glu Asn Asn His Arg Ile Phe Leu Ala
   530 535 540
   Gly Asp Lys Asp Asn Val Ile Asp Gln Ile Glu Lys Gln Ala Lys Asp
   545 550 555 560
   Leu Ala Phe Pro Gly Ser Gly Glu Gln Val Glu Lys Leu Ile Lys Asn
   565 570 575
   Gln Lys Glu Ser His Phe Val Ser Ala Arg Pro Gln Ser Gln Ser Gln
   580 585 590
   Ser Pro Ser Ser Pro Glu Lys Glu Ser Pro Glu Lys Glu Asp Gln Glu
   595 600 605
   Glu Glu Asn Gln Gly Gly Lys Gly Pro Leu Leu Ser Ile Leu Lys Ala
   610 615 620
   Phe Asn
   625
<210> 13
   <211> 614
   <212> PRT
   <213> Arachis hypogaea
<400> 13
   Met Arg Gly Arg Val Ser Pro Leu Met Leu Leu Leu Gly Ile Leu Val
   1               5                   10                  15
   Leu Ala Ser Val Ser Ala Thr Gln Ala Lys Ser Pro Tyr Arg Lys Thr
               20                  25                  30
   Glu Asn Pro Cys Ala Gln Arg Cys Leu Gln Ser Cys Gln Gln Glu Pro
           35                  40                  45
   Asp Asp Leu Lys Gln Lys Ala Cys Glu Ser Arg Cys Thr Lys Leu Glu
       50                  55                  60
   Tyr Asp Pro Arg Cys Val Tyr Asp Thr Gly Ala Thr Asn Gln Arg His
   65                  70                  75                  80
   Pro Pro Gly Glu Arg Thr Arg Gly Arg Gln Pro Gly Asp Tyr Asp Asp
                   85                  90                  95
   Asp Arg Arg Gln Pro Arg Arg Glu Glu Gly Gly Arg Trp Gly Pro Ala
               100                 105                 110
   Glu Pro Arg Glu Arg Glu Arg Glu Glu Asp Trp Arg Gln Pro Arg Glu
           115                 120                 125
   Asp Trp Arg Arg Pro Ser His Gln Gln Pro Arg Lys Ile Arg Pro Glu
   130 135 140
   Gly Arg Glu Gly Glu Gln Glu Trp Gly Thr Pro Gly Ser Glu Val Arg
   145 150 155 160
   Glu Glu Thr Ser Arg Asn Asn Pro Phe Tyr Phe Pro Ser Arg Arg Phe
   165 170 175
   Ser Thr Arg Tyr Gly Asn Gln Asn Gly Arg Ile Arg Val Leu Gln Arg
   180 185 190
   Phe Asp Gln Arg Ser Lys Gln Phe Gln Asn Leu Gln Asn His Arg Ile
   195 200 205
   Val Gln Ile Glu Ala Arg Pro Asn Thr Leu Val Leu Pro Lys His Ala
   210 215 220
   Asp Ala Asp Asn Ile Leu Val Ile Gln Gln Gly Gln Ala Thr Val Thr
   225 230 235 240
   Val Ala Asn Gly Asn Asn Arg Lys Ser Phe Asn Leu Asp Glu Gly His
   245 250 255
   Ala Leu Arg Ile Pro Ser Gly Phe Ile Ser Tyr Ile Leu Asn Arg His
   260 265 270
   Asp Asn Gln Asn Leu Arg Val Ala Lys Ile Ser Met Pro Val Asn Thr
   275 280 285
   Pro Gly Gln Phe Glu Asp Phe Phe Pro Ala Ser Ser Arg Asp Gln Ser
   290 295 300
   Ser Tyr Leu Gln Gly Phe Ser Arg Asn Thr Leu Glu Ala Ala Phe Asn
   305 310 315 320
   Ala Glu Phe Asn Glu Ile Arg Arg Val Leu Leu Glu Glu Asn Ala Gly
   325 330 335
   Gly Glu Gln Glu Glu Arg Gly Gln Arg Arg Arg Ser Thr Arg Ser Ser
   340 345 350
   Asp Asn Glu Gly Val Ile Val Lys Val Ser Lys Glu His Val Gln Glu
   355 360 365
   Leu Thr Lys His Ala Lys Ser Val Ser Lys Lys Gly Ser Glu Glu Glu
   370 375 380
   Asp Ile Thr Asn Pro Ile Asn Leu Arg Asp Gly Glu Pro Asp Leu Ser
   385 390 395 400
   Asn Asn Phe Gly Arg Leu Phe Glu Val Lys Pro Asp Lys Lys Asn Pro
   405 410 415
   Gln Leu Gln Asp Leu Asp Met Met Leu Thr Cys Val Glu Ile Lys Glu
   420 425 430
   Gly Ala Leu Met Leu Pro His Phe Asn Ser Lys Ala Met Val Ile Val
   435 440 445
   Val Val Asn Lys Gly Thr Gly Asn Leu Glu Leu Val Ala Val Arg Lys
   450 455 460
   Glu Gln Gln Gln Arg Gly Arg Arg Glu Gln Glu Trp Glu Glu Glu Glu
   465 470 475 480
   Glu Asp Glu Glu Glu Glu Gly Ser Asn Arg Glu Val Arg Arg Tyr Thr
   485 490 495
   Ala Arg Leu Lys Glu Gly Asp Val Phe Ile Met Pro Ala Ala His Pro
   500 505 510
   Val Ala Ile Asn Ala Ser Ser Glu Leu His Leu Leu Gly Phe Gly Ile
   515 520 525
   Asn Ala Glu Asn Asn His Arg Ile Phe Leu Ala Gly Asp Lys Asp Asn
   530 535 540
   Val Ile Asp Gln Ile Glu Lys Gln Ala Lys Asp Leu Ala Phe Pro Gly
   545 550 555 560
   Ser Gly Glu Gln Val Glu Lys Leu Ile Lys Asn Gln Arg Glu Ser His
   565 570 575
   Phe Val Ser Ala Arg Pro Gln Ser Gln Ser Pro Ser Ser Pro Glu Lys
   580 585 590
   Glu Asp Gln Glu Glu Glu Asn Gln Gly Gly Lys Gly Pro Leu Leu Ser
   595 600 605
   Ile Leu Lys Ala Phe Asn
   610